# EUROPEAN PATENT SPECIFICATION

(11) **EP 0 704 528 B1**
(45) Date of publication and mention of the grant of the patent: **01.06.2005**
(21) Application number: 95304719.8
(22) Date of filing: 05.07.1995
(51) Int. Cl.: C12N 15/41, A61K 39/125, C07K 14/08, C12N 15/86

(54) **Rabbit hemorrhagic disease virus (RHDV) recombinant capsids and proteins, diagnostic kits and vaccines containing them**
Hämorrhagie-Virus aus Kaninchen (RHDV), rekombinante Hullen und dessen Proteine, Diagnosesätze und Impfstoffe die sie enthalten
Virus de l'hémorragie des lapins (RHDV), capsides recombinants, protéines, et trousse de diagnostic et vaccins les contenant

(30) Priority: 08.07.1994 ES 9401493
(43) Date of publication of application: 03.04.1996
(73) Proprietor: Wyeth Farma, S.A., 28700 San Sebastian de los Reyes, Madrid (ES)
(72) Inventor: Plana Duran, Juan, E-17813 Vall de Bianya, Olot-(Gerona) (ES); Climent Sanchez, Isabel, E-17813 Vall de Bianya, Olot-(Gerona) (ES); Casal Alvarez, Jose Ignacio, E-28037 Madrid (ES); Rodriguez Garcia, Maria José, E-28037 Madrid (ES)
(74) Representative: Mannion, Sally Kim, Dr.

(56) References cited:
- EP-A- 0 551 449
- EP-A- 0 704 529
- FR-A- 2 690 838
- MEYERS G. ET AL.: "Rabbit Hemorrhagic Disease Virus - Molecular Cloning and Nucleotide Sequencing of a Calicivirus Genome." VIROLOGY, vol. 184, 1991, ORLANDO, US, pages 664-676, XP002049969
- PARRA F. AND PRIETO M.: "Purification and Characterization of a Calicivirus as the Causative Agent of a Lethal Hemorrhagic Disease in Rabbits." JOURNAL OF VIROLOGY, vol. 64, no. 8, 1990, US, pages 4013-4015, XP002049971
- PARRA F. ET AL.: "The amino terminal sequence of VP60 from rabbit hemorrhagic disease virus supports its putative subgenomic origin." VIRUS RESEARCH, vol. 27, 1993, AMSTERDAM, NL, pages 219-228, XP002051164
- BISHOP D.H.L.: "Baculovirus expression vectors" SEMINARS IN VIROLOGY, vol. 3, 1992, ORLANDO, US, pages 253-264, XP002049970
- ROITT I.M. & DELVES P.J.: "ENCYCLOPEDIA OF IMMUNOLOGY, VOL I" 1992 , ACADEMIC PRESS , LONDON, GB XP002050130 194140 * page 28 - page 30 *
- ROITT I.M. & DELVES P.J.: "ENCYCLOPEDIA OF IMMUNOLOGY, VOL III" 1992 , ACADEMIC PRESS , LONDON, GB XP002050131 194160 * page 1540 - page 1544 *

## Description

### FIELD OF THE INVENTION

This invention relates to recombinant capsids and proteins of the virus causative of rabbit hemorrhagic disease (RHDV), produced by expression in insect cells using a system of recombinant baculoviruses. The invention also relates to diagnostic kits and vaccines comprising the said recombinant capsids and proteins.

### BACKGROUND OF THE INVENTION

Early in the summer of 1988, the appearance of a strange disease was described in Spain, in rural rabbit farms of the zones of Asturias and Almeria-Murcia, in which breeders, and occasionally adult rabbits, died without any apparent clinical signs. Originally, this was attributed to a toxic type affection because of its acuteness, or to the myxomatosis virus as in those sick animals anti-myxomatosis virus antibodies were detected. These two hypotheses were discarded and it was concluded that the clinical signs were closely related to a contagious disease described in China (Jiangsu Province) for the first time in 1984 and designated "Hemorrhagic Rabbit Disease" or "Infectious Viral Pneumonia" (Liu, S.J., et al., 1984, An. Hus. Vet. Med., 16(6):253-255).

According to the data presented by Xu et al. in 1988 at the World Rabbit Congress held in Hungary (4th World Rabbit Congress W.R.S.A., 1988, 3: 456-462), this disease was introduced into China through an imported batch of Angora rabbits from the Federal Republic of Germany. This fact has given rise to the idea that this virus may have been latent in Europe for a long time. The first case of viral hemorrhagic disease in Europe was detected in Italy in 1986 and, at the first, denominated as "X Disease" (Cancelloti, F. M. et al., 1988, Coniglicoltura, 25, 9:41-46). Between 1988 and 1989 this disease was also described in France, Germany, and Spain (Villares, A., et al., 1988, Med. Vet., 5:645-650; Plana, J., et al., 1989, Med. Vet., 6:87-88).

The disease affects chiefly adult rabbits. Young sucking rabbits and fattening rabbits seem to be naturally protected. Animals from industrial farms seem to be less susceptible to the virus than those bred in small farms.

The period of incubation is 2-3 days; the mortality rate is higher than 90%. The clinical signs are convulsions, ataxia, dyspnea and sudden deaths with epistaxis. Lesions are characterized by generalized congestion and petechiae or hermorrhagic zones in different organs - mainly in the lungs. Different degenerative phases in liver are dectected, as this is a target organ for virus replication.

The causative agent of the disease is the virus denominated as Rabbit Hemorrhagic Disease Virus. This virus was isolated for the first time and simultaneously in Spain (Plana, J., et al., as quoted above; Parra, F. and Prieto, M., 1990, J. Virology, 64:4013-4015) and Germany (Ohlinger, V.F. et al., 1990, J. Virology, 64: 3331-3336). RHDV is 40 nm in size, non-enveloped with a single RNA chain as genomic material. Based on morphological data, it was initially classified as a picornavirus (Pu, B. et al. 1985, Chi. J. Vet. Med. 11:16-17) and parvovirus (Xu et al., 4th World Rabbit Congress, supra). However, based on the information corresponding to virion size, density, morphology and molecular structure, it has been definitively classified as a calicivirus.

The molecular characteristics and nucleotide sequence of a German RHDV isolate have been described in two publications:
a) Meyers, G., Wirblich, C., and Thiel, H-J., 1991, "Rabbit hemorrhagic disease virus. Molecular cloning and nucleotide sequencing of a calicivirus genome", Virology, 184:664-676.
b) Meyers, G., Wirblich, C., and Thiel, H-J., 1991, "Genomic and subgenomic RNAs of rabbit hemorrhagic disease virus are both protein-linked and packaged into particles", Virology, 184:677-686.

Recently, the complete RHDV genome was cloned and sequenced (Meyers, G., et al., supra). The RHDV genome is made up of a single strand RNA molecule containing at 3' end an adenine residue tail (poly-A). The length of the genome is approximately 800 nucleotides or base pairs (bp), and in its structure it contains a single open reading frame (ORF) which represents 89% of the viral genome. Region 5' codes for non-structural viral proteins, presenting a high level of homology in comparison with those coded by feline calicivirus (FCV). In this end, sequences similar to those described for picornavirus gene 2C involved in RNA synthesis, structures similar to those present in proteases containing cystein residues, and RNA dependent RNA-polymerase have also been described. On the contrary, region 3' of this single RHDV ORF codes for the structural protein that makes up the virus capsid. This region has been denominated as VP60 on account of its size, approximately 60 KDa, and its synthesis is produced both from a long transcript from the genomic RNA and posterior processing, as well as from the translation of a 2200 pb subgenomic RNA. These two viral RNA forms -characteristic of other RNA viruses- have been described for RHDV (Meyers, G. et al., supra), and it has been proposed that both are present in the virion associated to a 15 KDa protein and capsulated.

One of the main disadvantages for the preparation of a vaccine against the disease caused by RHDV is the fact that it has only been possible to replicate this virus in vivo. Some permissible cell systems have been described. Specifically, at our laboratories, we have grown the virus in rabbit kidney RH-13 cells, although with very low virus production. This means that, in order to obtain vaccine antigens, it is necessary to infect and sacrifice rabbits.

The present invention provides an alternative route for the solution of the disadvantages associated with a conventional vaccine. This alternative consists of preparing and using recombinant vaccines capable of effectively protecting against infection caused by RHDV. The new recombinant vaccines can contain the RHDV recombinant capsids and/or proteins provided by this invention. These recombinant vaccines do not require the use of complete virus, but only a part of it, eliminating the risk of a virus-releasing accident, obtaining a considerable advantage over conventional, first-generation vaccines. On the other hand, a new method for the diagnosis of RHDV with enzymatic immunoassay techniques (ELISA) has been developed using the recombinant capsids and/or proteins of the present invention.

Many systems for the expression and production of recombinant proteins are known. One of the most effective systems for large-scale production of recombinant proteins is based on the replication of recombinant baculoviruses derived from *Autographa californica* nuclear polyhedrosis virus (AcNPV), in insect cells in culture. The description of the expression technique in baculovirus is described in, among others, the following articles:
a) LucKow, V.A. and Summers, M.D., "Trends in the development of baculovirus expression vectors". Bio/Technology, 6:47-55, (1988).
b) Bishop, D.H.L., "Baculovirus expression vectors", Seminars in VIROLOGY, 3:253-264 (1992).

This invention provides RHDV recombinant capsids and proteins produced by means of a suitable expression system replicated on permissive host cells. In one particular case, the recombinant capsids and proteins provided by this invention were produced by expression in insect cells using a recombinant baculovirus system. The recombinant baculovirus capable of producing such recombinant capsids and proteins, as well as the transfer vector utilized, constitute two additional objectives of the present invention. The procedures for the obtainment of the said recombinant baculoviruses and products (capsids and proteins) also constitute an objective of the present invention.

The invention also provides a new vaccine capable of protecting rabbits against infection caused by RHDV, comprising the recombinant capsids and/or proteins provided by the present invention, and a suitable carrier or adjuvant.

In addition, the present invention provides a bi- or multivalent vaccine capable of preventing rabbit hemorrhagic disease and other rabbit infection or infections, comprising the RHDV recombinant capsids and/or proteins provided by the present invention, together with one or more rabbit pathogens and a suitable carrier or adjuvant.

The invention also provides a method and a diagnostic kit for the detection of the presence of antibodies that specifically recognize RHDV in biological sample -e.g., serum from rabbits suspect of being infected- comprising the use of recombinant capsids and/or proteins provided by the present invention and suitable detection methods.

The invention also provides a method and diagnostic kit for the detection of the presence of antigen (RHDV) in a biological sample -e.g. blood, serum, lung, spleen or liver- from rabbits suspect of being infected, comprising an antibody that specifically recognizes RHDV, having been obtained by means of immunizing animals with the recombinant capsids and/or recombinant proteins provided by the present invention and adequate detection methods.

### BRIEF DESCRIPTION OF THE FIGURES

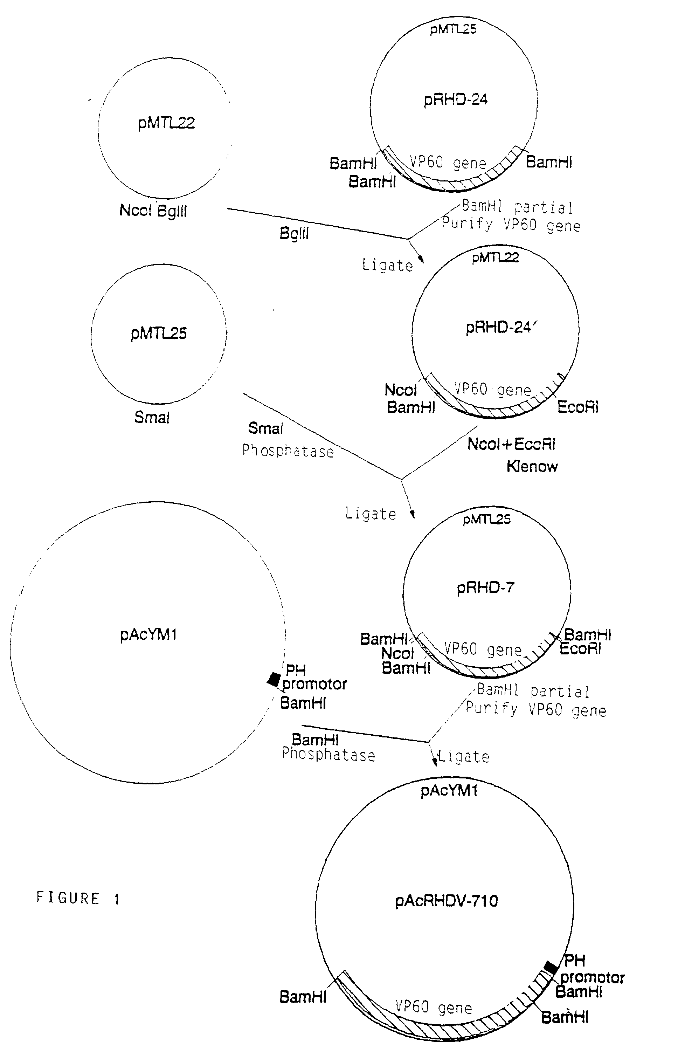

Figure 1 shows the construction of transfer vector pAcRHDV-710, indicating the manipulations done to insert the gene coding for the RHDV VP60 in plasmid pAcYM1.

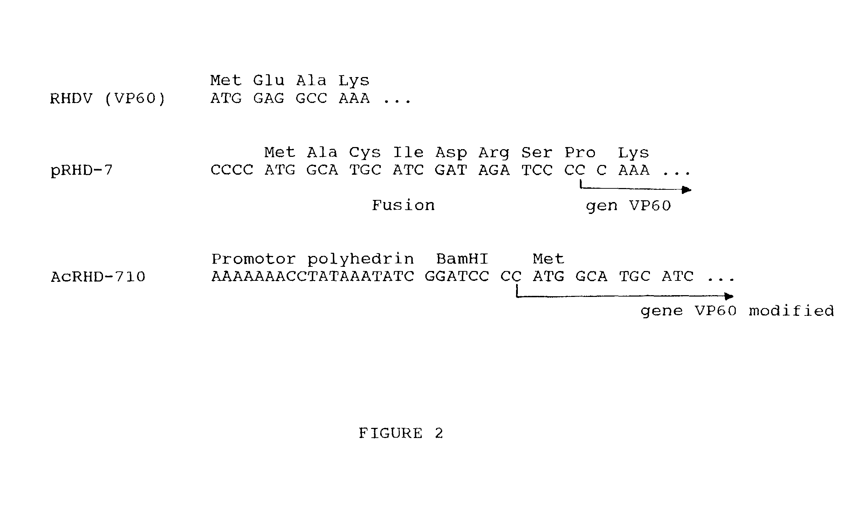

The native VP60 gene [RHDV(VP60)] and the modified gene [pRHD-7] structures appear in Figure 2, showing the differences that exist between both of them. It can be observed that, in this specific case, the modified gene contains a sequence coding for: (i) two amino acids, different from those present in the native VP60 sequence (sites +2 and +3 amino acids), which have been substituted in the recombinant protein by two other amino acids, and (ii) five additional amino acids that are not present in the native VP60 sequence and that originate in the sequence coding for the pMTL22 plasmid polilinker. This Figure also shows the correct orientation of the modified gene, in relation to the polyhedrin promoter in the recombinant pAcRHDV-710 clone, obtained by means of mapping with restriction endonucleases, where it can be observed that the distance between site *BamHI* and the beginning of transcription is of only 2 bases.

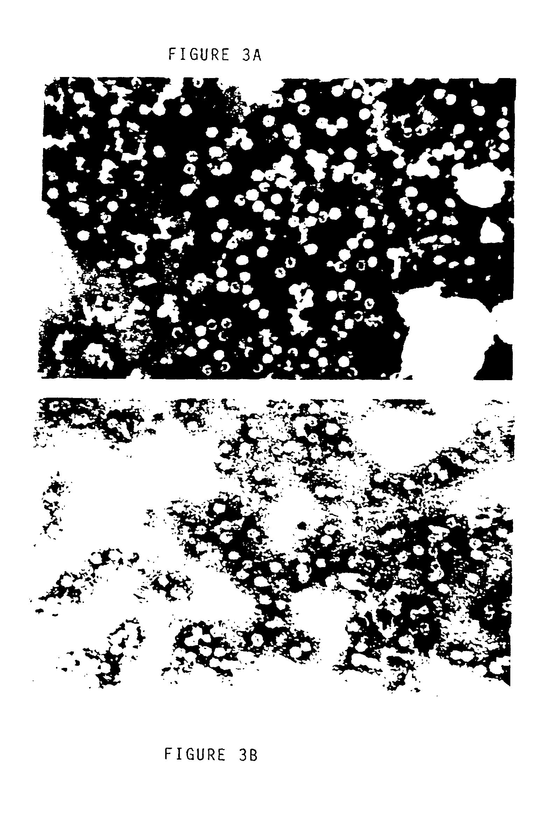

Figure 3 shows a preparation of viral capsids (Figure 3a) formed by the aggregation of one RHDV recombinant protein provided by this invention and a preparation of purified virions (Figure 3b).

### DESCRIPTION OF THE INVENTION

For a number of years, our Laboratory conducted an investigation to identify the causative agent of rabbit hemorrhagic disease (RHDV). As a consequence of this research, RHDV was isolated and characterized (Plana et al., supra), and a conventional vaccine against the said infectious disease was developed, marketed with the brand name CYLAP HVD^{R}.

More recently, our research efforts have addressed the isolation and cloning of the genome of the said RHDV isolate, with the objective of developing new recombinant vaccines effective against the infection caused by RHDV. To that end, a segment of its genome has been cloned - corresponding to end 3' of the viral genome which codes for the structural virus protein denominated VP60, possibly involved in viral antigenicity and immunogenicity.

The present invention provides RHDV recombinant capsids and proteins that are immunogenically and antigenically analogous to natural RHDV and to the native VP60 protein of this virus. For this reason, these products -recombinant capsids and/or recombinant proteins-can be used to manufacture vaccines capable of preventing the infection caused by RHDV, and diagnostic kits appropriate for this virus. The said recombinant capsids are obtained by means of aggregation of the RHDV recombinant proteins provided by the present invention.

The expression "antigenically analogous to natural RHDV and to the native VP60 protein of this virus", or similar, should be understood, in the sense used in the present description, to mean that the recombinant capsids and/or recombinant proteins are capable of inducing production of antibodies in the same way as is induced by natural RHDV and the native VP60 protein of this virus.

The expression "immunogenically analogous to natural RHDV and to the native VP60 protein of this virus", or similar, should be understood, in the sense used in the present description, to mean that the recombinant capsids and/or recombinant proteins are capable of inducing an immune response in an animal sufficient to protect it against an infection caused by RHDV, and for this reason the aforesaid recombinant capsids and/or recombinant proteins are suitable for use in the formulation of RHDV vaccines.

The recombinant capsids and proteins provided by the present invention can be produced in a suitable expression system replicated on appropriate cells, by means of using conventional Genetic Engineering techniques.

In a specific realization of the present invention - which will serve as a detailed illustration of one way in which it can be carried out- a recombinant protein comprising the entire amino acid sequence of the RHDV native VP60 protein, except for two sites +2 and +3 amino acids, was cloned and expressed. This recombinant protein is denominated in the present description as "recombinant VP60", "rVP60", or "modified VP60".

The recombinant VP60 protein comprises the RHDV native VP60 amino acid sequence, with the following modifications:
(i) sites +2 and +3 amino acids of the original native VP60 protein sequence have been subsituted by two other amino acids in the recombinant VP60 protein, and
(ii) the recombinant VP60 protein includes a sequence of five additional amino acids, located at the amino terminal end which is not present in the native VP60 protein sequence. The said sequence of five additional amino acids originates from the sequence coding for the pMTL22 plasmid polilinker. These five amino acids are nonfunctional and, alternatively, the said additional sequence could be substituted by another analogous sequence of equal or different number, without altering the final result.

Other characteristics of the said recombinant VP60 protein, which will be described in more detail further down, are the following:
a) it has been produced in a recombinant baculovirus expression system replicated on insect cell culture;
b) it is capable of, surprisingly, forming capsids or multimeric structures or proteinaceous aggregates similar to RHDV viral capsids; and
c) the recombinant VP60 protein as well as the capsids or proteinaceous aggregates that they may form are immunogenically and antigenically analogous to the RHDV native VP60 protein and to the native virus, for which reason they are appropriate for the immunizing of rabbits against infection caused by RHDV, and can be used:
   - in the formulation of active, passive, uni-, bi- or multivalent vaccines capable of protecting rabbits from infection caused by RHDV and other rabbit pathogens; and
   - in the preparation of diagnostic kits for the detection of the presence (i) of antibodies that specifically recognize RHDV or (ii) of RHDV by means of using antibodies that specifically recognize RHDV obtained by immunization of animals with recombinant capsids and proteins provided by the present invention.

As described in more detail below, in a specific realization of this invention, RHDV recombinant capsids and proteins were produced, specifically the protein denominated recombinant VP60, in a recombinant baculovirus expression system multiplied in permissive host cell culture. The global procedure for the obtainment of the aforesaid recombinant VP60 protein comprises, basically, the following general stages:
I. Preparation of the cDNA sequence to be inserted in a baculovirus; and
II. Obtainment of recombinant baculoviruses expressing the recombinant proteins.

These general stages are subdivided into other substages. Thus, the preparation of the cDNA sequence to be inserted comprises the substages of:
I.a Isolation and purification of RHDV;
I.b Isolation of its total viral RNA; and
I.c Synthetization of the cDNA from genomic RNA.

The obtainment of a recombinant baculovirus, expressing the RHDV recombinant VP60, comprises the following substages:
II.a Preparation of the gene coding for the RHDV VP60;
II.b Insertion of the said gene in a baculovirus transfer vector;
II.c Transfection of permissive host cells with the said transfer vector which has the gene coding for the RHDV VP60 inserted; and
II.d Selection of the recombinant baculoviruses expressing the said recombinant VP60.

Afterwards, the characterization of the recombinant baculoviruses as well as the characterization and purification of the obtained recombinant proteins is carried out. All these stages are described in more detail further down.

The procedure for the obtainment of RHDV recombinant capsids and proteins starts with the isolation and purification of RHDV, in this case from an isolate available at our laboratories, in accordance with the protocol described in Example 1. Once the virus had been isolated and purified, the total viral RNA was isolated by means of treating the sample with SDS (sodium dodecyl sulfate), pronase and K proteinase and extractions with phenol:chloroform (Example 2). The RNA obtained was analyzed in neutral agarose gels at 0.7% by staining with ethidium bromide, and two majority bands were observed which could correspond to the genomic and subgenomic RNA of the virus. The comparison between these bands with RNA standards of known sizes allowed to deduce a size of 8 Kb for one of the bands.

Afterwards, the cDNA corresponding to end 3' of the viral RNA was synthesized (Example 3) with a commercial kit (BOEHRINGER), by using a strategy that takes advantage of the presence of poly(A) tails to use the oligo d(T) as extension primer capable of being extended with reverse transcriptase enzyme and synthesizing cDNA molecules. cDNA synthesis was verified and quantitated by means of counting the radioactivity incorporated in the synthesized material, and electrophoresis in alkaline and neutral agarose gels.

For the cloning of cDNA, in the first place, a size selection of the synthesized cDNA was done. cDNA fragments of three sizes were selected, i.e.: between 1,000 and 2,000 bp, between 2,000 and 5,000 bp, and over 5,000 bp. The purified cDNA was cloned in blunt ends in vector pMTL25, linearized with *SmaI*, and dephosphorylated with alkaline phosphatase. The insert-vector ligation was done with DNA ligase, and it was used to transform *E. coli* XL-1Blue cells that allow to carry out the initial color-based selection of recombinant colonies.

The analysis of the positive clones was done by means of plasmidic DNA preparations and mapping of restriction sites with the *BamHI* and *EcoRI* enzymes, based on the sequence of a German RHDV isolate (Meyers et al., supra). Of the 38 plasmids analyzed, only 10 were positive and corresponded to RHDV zone 3', with inserts between 800 and 2,000 pb. Three of them, denominated pRHD-24, pRHD-25 and pRHD-45, were approximately 2,000 pb in size, and thus were more likely to contain the VP60 structural protein gene. For this reason, they were sequenced to verify authenticity and determine insertion zones, orientation and exact size, by means of the dideoxy technique applied to double-stranded plasmids. Universal oligonucleotides (5'GTAAAACGACGGCCAGT3') and reverse (5'AACAGCTATGACCATG3') were used. It was observed that clones pRHD-24 y pRHD-25 began at nucleotide 5313 (in accordance with the Meyers et al. sequence, supra) and that clone pRHD-45 began at nucleotide 5193. The first two clones ended at poly(A) tail located at end 3'.

To identify the VP60 protein initiation codon the following two strategies were followed:
(a) an experimental approximation, with the intention of sequencing the N-terminal end directly in an automatic protein sequencer, and
(b) a theoretical prediction by sequence analysis with the Microgenie computer program (BECKMANN).

No practical results were obtained from the direct sequencing of the protein, whereas the beginning of the VP60 coding sequence in nucleotide 5305 of the RHDV genome -taken as reference for future VP60 expression experiments- was calculated by means of simulating the sizes of possible transcript regions.

For the obtainment of recombinant baculoviruses expressing RHDV VP60, the following procedure was followed: First, the VP60 gene to be inserted was prepared. To that end, the plasmid denominated pRHD-24 in which the cDNA insert began 5pb below the theoretical ATG initiation codon was selected, for which reason it was necessary to add an ATG that would enable to begin expression. The complete pRHD-24 insert was obtained by partial digestion with *BamHI* and recloning at site *BglII* of the pMTL22 vector, so that the open reading frame of the insert remained in frame with the ATG belonging to the *pMTL22* target *NcoI* sequence. The construction obtained was digested with *NcoI* and *EcoRI* to eliminate the noncloning 3' region of the cDNA clone, polished with the Klenow fragment of *E. coli* DNA polymerase I, and recloned in pMTL25 digested with *SmaI*, resulting in a plasmid denominated pRHD-7 (Figure 1). By means of this construction, the original amino acids located at theoretical site +2 and +3 of native VP60 are lost, and these two amino acids (Glu and Ala) present in the native VP60 sequence are substituted by two other amino acids (Ser and Pro) present in recombinant VP60. The insertion of a sequence coding for five amino acids (Ala-Cys-Ile-Asp-Arg) -not present in the native VP60 protein sequence and which originate from the plasmid pMTL22 polilinker coding sequence- is also produced. These five amino acids are nonfunctional and, alternatively, the said additional sequence could be substituted by another analogous sequence of the same or different number, without altering the final result. The resulting plasmid (pRHD-7) was purified by alkaline lysis and characterized by mapping with restriction endonucleases and sequencing of the insertion regions. This plasmid was used in the extraction of modified VP60.

Afterwards, the gene coding for the modified VP60 was inserted into a suitable transfer vector -like the pAcYM1 vector which has a single *BamHI* site in line with the polyhedrin promoter. The modified VP60 gene had a *BamHI* restriction site in its sequence and, for this reason, it was difficult to clone the said pAcYM1 vector as it was necessary to make new partial digestions that would enable the extraction of the complete insert.

For the isolation of the VP60 gene from the pRHD-7 plasmid, different partial digestion times with *BamHI* were tested. It was observed that the most suitable times were between 15 and 30 minutes. By successive electrophoresis in agarose gel (Example 5) a 1.7 Kb band was collected and then ligated to plasmid pAcYM1, which had been digested with *BamHI* and dephosphorylated with alkaline phosphatase. With this ligation mixture *E.coli* DH5α cells were transformed and recombinant pAcRHDV-710 and pAcRHDV-709 plasmids (transfer vectors) were selected by mapping with restriction endonucleases. Both plasmids were sequenced to verify suitable insert orientation in relation to the polyhedrin promoter. It was observed that there was a distance of only 2 bases between the *BamHI* site and the initiation site of transcription.

Afterwards, *Spodoptera frugiperda* cells, Sf9 clone, were transfected with mixtures of parenteral AcRP23-lacZ virus purified infective DNA and the corresponding transfer vector. Once this cotransfection had been done, the recombinant baculoviruses were identified by plaque color phenotype assay, after the staining of the viral progeny with X-gal, and then purified. The recombinant baculovirus, denominated AcNPV RHDV-710, was deposited at the European Collection of Animal Cell Cultures (ECACC).

Examples 4 to 6 describe in detail the obtainment of recombinant baculoviruses expressing RHDV modified VP60.

Recombinant baculoviruses AcNPV RHDV-710 and AcNPV RHDV-709 expression products in Sf9 cells have been evaluated and the recombinant VP60 has been duly characterized by electrophoresis in SDS-PAGE gels and Immunoblotting.

By means of electrophoresis in SDS-PAGE gels, a 60 KDa protein, migrating in the same position as purified RHDV, was mainly obtained. It was also observed that the recombinant protein remained chiefly in the cell lysis supernatant, indicating that the protein was very soluble. If the gel was run for a sufficient period of time, it was possible to observe that the recombinant protein presented a motility slightly lower than viral motility. This was due to the in-frame fusion of the five additional amino acids.

It was verified by Immunobloting that the protein observed in the gel corresponded to the RHDV native VP60. To that end, the gel was transferred to a nitrocellulose membrane and the bands were blocked with skimmed powder milk and faced against polyclonal anti-RHDV rabbit serum (Laboratorios Sobrino). The result was conclusive, as the polyclonal anti-RHDV rabbit serum recognized the same 60 KDa protein both in cells infected with the recombinant AcNPV RHDV-710 baculovirus and in the positive control. However, it did not recognize any protein in the uninfected Sf9 extract (Example 7.2).

Later studies on the VP60 protein expressed by the AcNPV RHDV-710 recombinant baculovirus made it evident that, surprisingly, this protein was capable of forming capsids or multimeric structures or proteic aggregates similar to the viral capsids. For that reason, recombinant VP60 preparations were examined by electron microscopy and their hemagglutinating activity studied. Following the protocol described in Example 8, recombinant capsids were obtained, with a purity level higher than 80%, by precipitation with low ammonium sulfate concentrations.

The electron microscopy analysis of the recombinant VP60 samples revealed, surprisingly, the existence of proteic aggregates (capsids) structurally and morphologically similar to viral capsids. The size of these recombinant capsids is similar to that of the original virions (35-40 nm). Likewise, it can be observed that in the preparation of recombinant capsids all the particles are empty, i.e., do not contain genomic material (Figure 3).

On the other hand, the recombinant capsids were subjected to a hemagglutination assay with 1% human group O red blood cells in PBS, with positive HA results.

By means of ELISA technique, the behavior of the recombinant capsids against purified RHDV was compared. Following the protocol described in Example 8.3, it was observed that the anti-RHDV sera equally recognized both recombinant expression product (VP60 or VP60 capsid) as well as purifed virus. No reactivity in front of uninfected Sf9 cell extracts was manifested.

Based on all these results, it can be concluded affirming that the expression products (capsids and recombinant VP60 protein) are antigenically analogous to natural RHDV and to the RHDV native VP60 protein, and that the sequence introduced does not alter the protein's intrinsic capacity to form polymeric capsid-type structures.

The recombinant capsids and/or proteins of the present invention are capable of being used with diagnostic purposes, both for the detection of the presence of specific RHDV antibodies (Example 11) and for the detection of the presence of antigen (RHDV) by means of using antibodies that specifically recognize RHDV obtained by means of immunizing animals with the recombinant capsids and/or proteins of this invention.

Additionally, the aforesaid recombinant capsids and/or proteins can be used to immunize rabbits against RHDV. This enables their use in the formulation of recombinant vaccines capable of effectively protecting rabbits from infection caused by RHDV. These vaccines can be active or passive. Active immunization vaccines can be prepared by suspending such recombinant capsids and/or proteins in a suitable adjuvant. If such recombinant capsids and/or proteins were to be presented in freeze-dried form, they could be resuspended in an immunologically acceptable diluent or in an adjuvant. Passive vaccine -or more exactly, passive immunization-can be obtained by actively immunizing animals with such recombinant capsids and proteins and isolating polyclonal antibodies which, once isolated and purified, can be used as vaccinal therapy.

Phosphate-buffered saline (PBS) solutions or other similar solutions are immunologically acceptable diluents.

Any of the adjuvants commonly used in the formulation of vaccines may be used as adjuvant of the recombinant capsids and proteins, or as diluent. Thus, these adjuvants may be oily, based on mineral oils, glycerides and fatty ether-acid derivatives, synthetic and aqueous adjuvants, such as aluminum hydroxide, alumina gel suspensions, QuilA, MDP (muramyl dipeptide), ISCOM (Immuno Stimulant Complex) or liposomes.

Additionally, the present invention supplies bi- or multivalent vaccines capable of preventing rabbit viral hemorrhagic disease and another infection or other infections. These vaccines comprise a combination or association of the recombinant capsids and/or proteins provided by the present invention, together with one or more rabbit pathogens and a suitable carrier or adjuvant. Among those pathogens are the following: myxomatosis virus, Shope's Fibroma virus, *Bordetella bronchiseptica,* as well as species of the genera *Pasteurella, Clostridia, Salmonella, Staphylococcus, Haemophilus,* etc.

The vaccines provided by the present invention can be administered to the animal Intramuscularly (IM), Subcutaneously (SC), Intradermally (ID), and Orally. For IM or SC administration, the vaccine can be oily type [simple (W/O) and (O/W) or double (W/O/W)], aqueous, or another type, for example, using MDP, liposomes or ISCOM. For ID administration, any conventional administration system can be used, preferably the system with the tradename DERMOJET^{R}. The vaccine for Oral administration may or may not contain aqueous adjuvant together with the antigen.

The form of presentation of the vaccinal antigen (recombinant product) can be in the form of a solution or suspension with one of the aforementioned antigens. In this presentation, diluents or other rabbit vaccines may be used. The presentation may also be in freeze-dried form, in which case the final product may be reconstituted with a diluent, an adjuvant or any other type of aqueous or oily vaccine for application in rabbits.

The vaccines provided by the present invention are suitable for the protection of wild, farm and pet rabbits against RHDV.

### DETAILED DESCRIPTION OF THE INVENTION (EXAMPLES)

The invention is described below in further detail, and as an Example, in relation with the obtainment of the aforementioned recombinant VP60 protein and the capsids that it can form, plus the vaccinal and diagnostic applications of the two recombinant products.

The achievement decribed below is meant only as an illustrative example of a specific way in which the present invention protein or capsid may be realized.

### Example 1. Obtainment and purification of RHDV.

### 1.1. Infection of rabbits with RHDV

### 1.1.1. Animals

Hybrid rabbits bred in our own farms -of approximately 3 months of age and weighing over 2 kg- were used. Previous to infection, the animals were bled by means of cardiac puncture, and the absence of anti-RHDV antibodies was verified by means of the analysis of the sera by the hemagglutination inhibition (HAI) test.

In brief, the HAI technique includes previous treatment of the serum to eliminate the components that inhibit hemagglutination. To that end, the serum was incubated at 56ºC for 30 minutes. To 0.1 ml of serum, 0.4 ml of phosphate-buffered saline solution (PBS) and 0.5 ml of Kaolin (SIGMA) suspension at 25% in PBS were added, maintaining for 1 hour at room temperature with frequent stirring. Once adsorption had been completed, the samples were centrifuged for 10 minutes at 200 g. To the supernatant, 50 µl of human group O red blood cells was added, and the suspension was stirred and centrifuged again as described above. The rest of the assay was done in sterile microtest plates with 96 U-shaped wells. To 50 µl of different dilutions of the treated serum were added 4 HAu (hemagglutination units) of RHDV. The plates were incubated for 30 minutes at room temperature, and afterwards, 50 µl of human group O red blood cell suspension at 1% in PBS were added. The reaction was incubated at 4ºC and monitored between 2 and 24 hours later.

### 1.1.2. Infection

A pathogenic RHDV isolate from our own laboratory was used (Plana, J., et al., 1988, Med. Vet., 6, 87-88). From this isolate (PV1-MSV1), three passages in rabbits were done, with P3 used as the inoculum in this infection. The rabbits were infected intranasally with 0.5 ml of an RHDV suspension in saline solution (20,400 HAu/animal). The virus was titrated by means of a hemagglutination assay on human group O red blood cells. To that end, 50 µl of a suspension of human group O red blood cells at 1% in PBS were mixed with 50 µl of the different dilutions of the virus in PBS. The absence or presence of agglutination in the red blood cells was evaluated at between 2 and 24 hours of incubation at 4ºC.

### 1.1.3. Extraction of livers

As a consequence of the infection, the rabbits died between 3 and 5 days post infection (d.p.i.). The liver and spleen were extracted from the dead animals. Tissue (fats, conjunctive tissue) and gallbladder remains were separated from the liver. Finally, they were washed with saline solution (PBS) and maintained at 4ºC until use.

### 1.2. Purification of RHDV

Virus purification was performed from the livers of rabbits infected with RHDV. The liver was cut into pieces, adding approximately equal volume of PBS. A hepatic tissue homogenate was prepared by means of an Ultraturrax, approximately 10 cycles of 2 minutes, with rest intervals, in ice bath. Later, the obtained suspension was clarified by means of centrifugation at 20,000 g and 4ºC for 35 minutes. The resulting supernatant was centrifuged for 2 hours at 113,000 g and 4ºC over sucrose cushion at 17% in PBS for the purification and sedimentation of the virus. Once the supernatant had been withdrawn, the precipitate was resuspended in PBS. Then, the virus suspension was extracted (1:1) with 1,1,2-trichlortrifluorethane (MERCK). The aqueous phase from a second extraction with solvent, containing the virus, was purified in a continuous sucrose gradient (15-30% w/v in PBS) by means of centrifuging at 113,000 g for 4 hours. Once centrifugation had been completed, the gradient was broken automatically into fractions (approximately 1 ml/ fraction), and the presence of virus in the different fractions was determined by hemagglutination (Example 1.1.2). The fractions corresponding to the two maximum agglutination peaks, denominated peak-I and peak-II, were joined, diluted with TE buffer (Tris-HCl 10 mM pH = 8.0 and EDTA 1 mM), and finally centrifuged for virus sedimentation at 113,000 g for 7 hours.

The analysis of the purified virus was done by electrophoresis in polyacrilamide-SDS (sodium dodecyl sulfate) gels at 9% (Laemmli, U.K., Nature, 227:680, 1970). Detection of total proteins was done by means of staining with Coomassie blue, and immunoblots (Towbin, H., et al., 1979, Proc. Natl. Acad. Sci. USA, 76: 4350-4354). The blots were developed using specific anti-RHDV serum and a peroxidase-protein A conjugate (SIGMA). A majority band of approximately 60 KDa was identified in peaks I and II of the gels stained with Coomassie blue. Later, it was confirmed that they correspond to the size described for the RHDV structural VP60 protein. In addition to this protein, a band of approximately 40 KDa was observed by means of Coomassie blue, more abundant at peak-II than at peak-I. The fact that this protein also reacted to anti-RHDV serum suggests that it is a partial degradation product of the 60 KDa protein.

### Example 2. Isolation of the viral RNA.

A purification was done of the total RNA of the virus sample, purified as described in Example 1.2. In brief, 200 µl of peak-I were treated with SDS at 1%, 0.5 mg/ml of K proteinase (SIGMA) and 2 mg/ml of pronase (SIGMA) at 37ºC for 30 minutes, to inactivate the proteins and RNases. Afterwards, the sample was treated with phenol two consecutive times with phenol:chloroform:iso-amyl alcohol to extract the RNA, which was finally precipitated by means of adding 1/10 volume of 3M sodium acetate, 0.25 mg/ml of glucogen, and 2.5 volumes of ethanol (1 hour at -20ºC). Once this period had elapsed, the RNA was recovered by centrifuging at 16,000 g and 4ºC for 30 minutes. After withdrawing the supernatant, the RNA precipitate was resuspended with 40 µl of TE.

The obtained RNA was analyzed in neutral agarose gels at 0.7% by staining with ethidium bromide. Two majority bands -which might correspond to the genomic and subgenomic RNA of the virus- were observed. The comparison of these bands with RNA standards of known sizes allowed to deduce an 8 Kb size for one of the bands. The presence of low molecular weight material in these gels must be pointed out, possibly corresponding to cellular DNA or RNA.

### Example 3. cDNA synthesis from the RHDV total RNA.

### 3.1. Preparation of the cDNA

The cDNA corresponding to end 3' of the RNA of RHDV was synthesized. This strategy takes advantage of the presence of poly(A) tails at end 3' in order to use the oligo d(T) as extension primer that can be extended with reverse transcriptase enzyme and can synthesize DNA molecule copies (cDNA).

cDNA synthesis was carried out with a commercial kit (BOEHRINGER) and was done, following the method briefly described below:
1 µg of the RHDV RNA-poly(A), obtained as described in Example 2, was incubated in the presence of 1 mM in dATP, dCTP (5-10 µCi of ³²P-α-dCTP), dGTP and dTTP, 25 RNase-inhibitting units, 0.8 µg of phosphorilated oligo d(T)₁₂ at end 5' and 40 units of reverse transcriptase, in a final volume of 20 µl. The reaction was incubated at 42ºC for 1 hour and then, in the same tube, synthesis of the second strand was started. To that end, buffer, RNase and 25 units of *E. coli* DNA polymerase were added, incubating at 22ºC for 1 hour, and at 65ºC for 10 minutes. Finally, to generate blunt ends, 4 DNA T4 polymerase units were added and, after 10 minutes at 37ºC, the reaction was stopped by adding EDTA (ethylene diamine tetraacetic acid) and sarcosil. Afterwards, the mixture was extracted with phenol:chloroform:iso-amyl alcohol, and the material precipitated with ethanol, as described in Example 2.

cDNA synthesis was confirmed and quantitated by counting the radioactivity incorporated into the synthesized material, and electrophoresis in alkaline and neutral agarose gels.

### 3.2. Cloning of the cDNA

First, the synthesized cDNA was size selected to avoid cloning excessively small segments. For that purpose, the material from the cDNA synthesis was recovered by centrifuging at 16,000 g for 30 minutes. The precipitate was vacuum-dried, dissolved in TE buffer and loaded in 1% agarose gel. cDNA fragments of three sizes were selected separately: of between 1,000 and 2,000 bp; between 2,000 and 5,000 bp; and larger than 5,000 bp. In all cases, material was recovered from the gel with DEAE-cellulose paper, elution with NaCl and subsequent precipitation. The purified cDNA was cloned in blunt ends in vector pMTL25 -a vector derived from pUC18. To that end, the vector was linearized with *SmaI* and treated with alkaline phosphatase in order to reduce the ligation background. After ligation with DNA T4 ligase, at 14ºC overnight, *E. coli* XL-1Blue competent cells which, in the presence of X-gal (5-bromo-4-indolyl-β-D-galactopyranoside) (BOEHRINGER) and IPTG (isopropyl-β-D-thiogalactopyranoside) (GOLD BIOCH), allow the initial selection of recombinant colonies by color (blue colonies without insert in comparison with white ones with insert).

The analysis of the positive clones was done by means of plasmid DNA preparations (Birnboim & Doly, 1979, Nucleic Acids Res., 7:1513-1523) and mapping of restriction sites with *BamHI* y *EcoRI* enzymes, based on the sequence of the German RHDV isolate (Meyers et al., supra). Out of the 38 plasmids analyzed -from the three corresponding size selections of cDNA and their cloning-only 10 were positive which corresponded to RHDV zone 3', with inserts between 800 and 2,000 bp. Three of them, denominated pRHD-24, pRHD-25 and pRHD-45, were approximately 2,000 bp in size and were therefore more likely to contain the VP60 structural protein gene, for which reason they were sequenced to confirm authenticity, determine the insertion zones, orientation and exact size. With this purpose, the dideoxy technique applied to double strand plasmids (Sanger, F., et al., 1977, Proc. Natl. Acad. Sci. USA, 74:5463-5467), and the universal (5'GTAAAACGACGGCCAGT3') and reverse (5'AACAGCTATGACCATG3') oligonucleotides were used. The analyzed RHDV clones were sequenced, resulting in the observation that clones pRHD-24 and pRHD-25 began at nucleotide 5313 (in accordance with the Meyers et al. sequence, supra) and clone pRHD-45 at nucleotide 5193. The three clones ended at the poly(A) tail located at end 3'.

### 3.3. Identification of the VP60 protein initiation codon

Because the VP60 protein is comprised within a single open reading frame comprising most of the viral genome, two strategies were followed in order to locate the position of the initial ATG corresponding to the VP60 gene. One strategy was to follow an experimental approach, trying to sequence the N-terminal end directly in an automatic protein sequencer; the other strategy was a theoretical prediction by means of sequence analysis with the computer program MICROGENIE (BECKMANN).

The direct sequencing of the protein did not give practical results as a clear sequence was not obtained. The reasons for this may be the presence of structural ends that block the amino end of the protein.

On the other hand, by means of simulating possible transcript regions sizes, the beginning of the VP60 protein coding sequence in nucleotide 5305 of the RHDV genome (Meyer et al., supra) was calculated. This nucleotide was used as reference for subsequent rVP60 expression experiments.

### Example 4. Preparation of the gene for its insertion into the transfer vector.

Based on the hypothesis established in Example 3.3, the cDNA clone which began closer to the said ATG was selected. None began immediately before and, for this reason, clone pRHD-24 was selected. In this clone, the cDNA insert began 5 bp below the theoretic initial ATG and therefore required the addition of an ATG that would allow the start of expression. The strategy followed is described below.

The complete insert of plasmid pRHD-24 was obtained by means of partial digestion with *BamHI* -in view of the presence of another internal *BamHI* site- and recloning at site *BglII* of the pMTL22 vector, in such a way that the insert's open reading frame is located in frame with the ATG belonging to the pMTL22 target *NcoI* sequence. Then, this construction was digested with *NcoI* and *EcoRI* -to eliminate the non-cloning 3' region of the cDNA clone-, treated with the Klenow fragment of *E. coli* DNA polymerase I (at room temperature for 15 minutes) in order to transform into blunt ends and, finally, recloned in pMTL25 digested with *SmaI*. The resulting plasmid was denominated clone pRHD-7 (Figure 1).

To summarize: With this construction, the original amino acids located at native VP60 theoretical +2 and +3 sites are lost, producing the substitution of these two amino acids (Glu and Ala) by two other amino acids (Ser and Pro) present in the modified VP60 protein, and the insertion of five amino acids (Ala-Cys-Ile-Asp-Arg), which are not present in the native VP60 sequence and originate from the pMTL22 sequence. The resulting plasmid, denominated pRHD-7, was used in the extraction of the modified VP60 gene.

### Example 5. Cloning in the pAcYM1 baculovirus transfer vector

Vector pAcYM1 (Matsuura et al., 1987, J. Gen. Virol., 68, 1233-1250) has a single cloning site - *BamHI* - in line with the polyhedrin promoter. As the modified VP60 gene has in its sequence one *BamHI* restriction site, cloning in the said vector is difficult because of the need to make new partial digestions that will allow complete insert extraction.

pAcYM1 has an advantage over other baculovirus transfer vectors: it does not use β-galactosidase as marker, an thus the only expression product obtained is from the gene introduced under the control of the polyhedrin promoter - in this particular case, recombinant VP60.

For the isolation of the VP60 gene from the pRHD-7 clone, different time lengths for partial digestion with *BamHI* were tested. In a first approach 0, 1, 2, 5 and 10 minute lengths were tested. Since not enough insert was released, longer periods were studied: 1, 2, 5, 10, 15, 20, 25 and 30 minutes. 10% from each digestion was loaded on agarose gel in order to observe the most suitable period. The suitable time periods proved to be 15, 20, 25 and 30 minutes. The rest of the digestion was loaded on a 1% agarose gel, on one side for 15 and 20 minutes and on the other for 25 and 30 minutes, in order to collect the 1.7 Kb band. The highest band (HB) and the band immediately below (LB) were collected with PIB-cellulose membranes (SCHLEICHER & SCHUELL). The DNA samples were eluted from the membranes in accordance with the manufacturer's instructions. The eluted material was precipitated with ethanol and resuspended in 30 µl volume of TE. To avoid a possible religation bottom of the parenteral pMTL25 vector, a previous total digestion with *Sca*I was done.

The material prepared this way was loaded on 1% agarose gel. Two bands appeared again in the eluted HB, corresponding to HB and a contamination by LB. The higher band was extracted again and ligation was done with pAcYM1 digested with *BamHI* and dephosphorilated with alkaline phosphatase. With this ligation mixture, *E.coli* DH5α was transformed and recombinant pAcRHDV-710 and pAcRHDV-709 clones were selected by mapping with restriction endonucleases. Both were sequenced (Figure 2), using the dideoxy technique (Sanger, 1977, supra), to confirm the correct orientation of the insert regarding the polyhedrin promoter. The distance between the *BamHI* site and the beginning of transcription was only 2 bases.

### Example 6. Transfection and obtainment of recombinant baculoviruses

Each of the transfer vectors, denominated pAcRHDV-710 and pAcRHDV-709, was used to cotransfect *Spodoptera* *frugiperda* insect cells, Sf9, with DNA of the AcRP23-LacZ parenteral baculovirus (donated by Dr. Posee, I.V.E.M., Oxford, U.K.), following Felgner's technique (Felgner et al., 1987, Proc. Natl. Acad. Sci. USA, 84, 7413-7417). For the cotransfection, a mixture of 2 µg of the transfer vector corresponding to pAcRHDV-709 or pAcRDHV-710 plus 500 ng of parenteral virus in presence of lipofectin (GIBCO-BRL) was used. The mixture was added to a monolayer with 2.5x10⁶ Sf cells in T25 flasks (COSTAR). The transfection mixture was withdrawn after 24 hours and TNMFH medium containing 5% fetal serum was added. The cells were incubated at 27ºC. The cell culture supernatant and cell remains were collected after 7 days. The infected cell culture supernatant was used to select the recombinant baculoviruses expressing the recombinant VP60 by successive platings in the presence of X-gal and neutral red. The recombinant baculoviruses gave rise to white plaques, whereas wild ones gave rise to blue plaques.

The recombinant baculoviruses obtained were denominated AcNPV RHDV-710 and AcNPV RHDV-709. Recombinant baculovirus AcNPV RHDV-710 was deposited at the European Collection of Animal Cell Cultures (ECACC) on 18th May 1994, with accession number V94051819.

### Example 7. Evaluation of the AcNPV RHDV-710 and AcNPV RHDV-709 expression products in Sf9 cells. Characterization of the recombinant VP60.

### 7.1. Electrophoresis in SDS-PAGE gels

Sf9 cells were infected with each recombinant baculovirus and collected at 72 h.p.i. (hours post infection). The cells were collected by centrifugation at 500 g for 5 minutes, then washed with phosphate buffered saline solution (PBS) pH:7.4 and resuspended at 10x10⁶ cells/ml in lysis buffer (containing 5% SDS, 1% β-mercaptoethanol and 17.4% glycerol). Before being analyzed in 9% SDS-PAGE gel, the extract was sonicated to separate the soluble fraction from the insoluble fraction. Simultaneously, samples were loaded with the peak I purified virus and with Sf9 uninfected extracts. The gels were stained with Coomassie blue. In the samples corresponding to the recombinant baculoviruses, a protein of approximately 60 KDa which migrated in position similar to that of the purified virus protein, appeared as majoritary protein. It was also observed that, mainly, recombinant protein remained in the cell lysis supernatant, indicating that the protein was a very soluble one.

If the gel is run for sufficient time, it can be observed that the recombinant protein presents a motility slightly lower than viral protein motility - due to the in-frame fusion of 5 extra amino acids.

### 7.2. Immunoblotting

An immunoblot test was conducted to confirm that the protein observed in the gel was the intended product (RHDV rVP60). The gel was transferred to a nitrocellulose membrane in a semidry device (BIORAD), in accordance with previously established protocols (Burnette et al., and Towbin et al., supra). The nitrocellulose bands were blocked with powdered skimmed milk (MOLICO^{R}, Nestlé) at 3% in buffer Tris-HCl 20mM pH:7.5, NaCl 500 mM [TBS] at room temperature for 1 hour. They were then mixed with a polyclonal anti-RHDV rabbit serum, diluted at 1:100 in TBS - 0.05% Tween-20, at room temperature for 2 hours, and then washed with TBS wash solution containing 0.05% of Tween-20 for 30 minutes. After incubation with protein A marked with peroxidase (dilution 1:1,000 in TBS-Tween-20) for 1 hour and subsequent washing, the bands were developed with 4-chloro-1-naphthol (SIGMA), 17% (v/v) methanol and 0.015% oxygenated water in TBS until visible bands appeared. The reaction was stopped with distilled water.

The result was conculsive. The polyclonal anti-RHDV rabbit serum recognized the same 60 KDa protein, both in cells infected with recombinant baculovirus AcNPV RHDV-710 and in the positive control (peak I). However, it did not recognize any protein in the uninfected Sf9 extract.

Bands, lower than the 60 KDa band recognized by the serum, appeared in both peak I and the AcNPV RHDV-710 extracts. Several explanations may be given for the presence of this extra band, although in no case is the quality of the final product affected, as demonstrated in the case of parvovirus, where they also appear.

In view of the fact that, of the two recombinant baculoviruses obtained, AcNPV RHDV-710 gave the best yield, this clone was selected for all subsequent experiments, including the preparation of the definitive inoculum deposited at ECACC (Example 6).

### Example 8. Analysis of the formation of empty capsids based on the RHDV recombinant VP60

In order to analyze if the recombinant VP60 protein expressed by the recombinant AcNPV RHDV-710 baculovirus was capable of forming multimeric structures similar to viral capsids, the recombinant VP60 preparations were inspected by electron microscopy and study of the hemagglutinating activity.

Sf9 cells were infected with AcNPV RHDV-710 at a multiplicity of 1 pfu (plaque forming unit) per cell and then incubated at 27ºC for 72 hours. The cells were collected by centrifugation, washed with PBS as described above, and resuspended at 2x10⁷ cells/ml in bicarbonate 25 mM pH: 9.5 solution in order to proceed with cell lysis. The lysed cell extract was centrifuged at 9,000 g for 15 minutes for the withdrawal of cell remains, and the supernatant was subjected to a precipitation with ammonium sulfate at 30% under standard conditions. Because of the small percentage of ammonium sulfate, only multimeric structures are expected to precipitate. The purity of the preparation was estimated by analysis in SDS-PAGE gels at 9%. It was confirmed that it is possible to obtain recombinant procapsids with purity level higher than 80% in a simple precipitation step.

### 8.1. Electron microscopy analysis

Semipurified recombinant VP60 samples were analyzed by electron microscopy with the appearance of proteic aggregates, structurally and morphologically similar to viral capsids. Figure 3a shows a preparation of purified capsids (increased x64k) and figure 3b shows peak II purified virions (increased x79k). The size of these recombinant procapsids is similar to the size of the original virions and can be estimated at about 35-40 nm. In the procapsid preparation, it can be observed that all the particles are empty, whereas in the virion preparation both types are present.

### 8.2. Hemagglutination test

The same recombinant VP60 samples used in Example 8.1 were tested by hemagglutination with human group O red blood cells at 1% in PBS. The titres obtained were:

| | |
|---|---|
| VP60 | 15,000 HAu/50 µl |
| RHDV (peak I) | 390,750 HAu/50 µl |

However, it must be taken into account that in VP60 the amount of protein is five times less than in peak I.

### 8.3. ELISA

By means of ELISA technique, the behavior of the recombinant procapsids in front of RHDV purification peak I was contrasted. A plaque assay was done on different antigen concentrations in carbonate buffer pH:9.6 at 4ºC (500, 100, 20 and 4 ng per well). They were washed three times and then mixed with three rabbit sera at dilutions of between 1/100 and 1/12,500, using factor 5 dilutions in 1% PBS-Molico^{R}. Incubation was done at 37ºC for 2 hours, washing three times and then adding protein A marked with peroxidase at 1:1,000 dilution in 1% PBS-Molico^{R}, at room temperature for 1 hour. Washing was done five times and color was developed using ABTS [2,2'-azino-bis(3-ethyl-benzothiazoline-6 sulfonic acid)] as substrate for 20 minutes. The reaction was stopped with 1% SDS and absorbance was monitored at 405 nm.

Optimal antigen concentration for the ELISA was estimated at between 500 and 100 ng per well.

Immediately afterwards, the sera were titrated using factor 2 dilution in 0.35 µg antigen/well, comparing peak-I and recombinant VP60. The test conditions were similar to those described above. The serum dilutions that distinguished best between positive and negative were 1/200 and 1/400.

Anti-RHDV sera equally recognize expression product (rVP60) as well as purified virus, presenting no reactivity against uninfected Sf9 cellular extracts.

Based on all these results, it can be concluded by affirming that the expression product (rVP60) is antigenically analogous to the RHDV native VP60 protein, and that the introduced sequence does not alter the intrinsic capacity of the recombinant protein to form polymeric capsid-type structures.

### Example 9. Vaccine Formulation

Vaccines -capable of protecting rabbits against challenge with RHDV- were prepared in the form of oily or aluminum hydroxide/Quil-A emulsion, following the procedure described below.

A 300 ml suspension culture of Sf9 cells was infected with recombinant baculovirus AcNPV-RHD710 at multiplicity of infection (m.o.i.) of 1 pfu/cell. The cells were collected at 72 h.p.i. and then processed as described above in Example 7, until the final step of purification of recombinant VP60 protein with ammonium sulfate at 30%. This sample was analyzed in SDS-polyacrilamide gels at 9% and immunoblot using an anti-RHDV serum. A majoritarian band (>80%) with molecular weight of approximately 60 KDa was observed, together with two smaller bands (43-60 KDa) that co-purify with VP60, although -not having been detected with anti-RHDV serum- they are not virus specific.

The recombinant VP60 protein concentration in the preparation was 2 mg/ml, determined by Lowry and SDS-gel with Coomasie stain.
**A.** An oily vaccine was formulated as follows:
a) 50% of antigenic phase, constituted of purified recombinant VP60 protein (rVP60) in PBS buffer; and
b) 50% of an oily phase, constituted of 82.3% Marcol-82 (ESSO ESPAÑOLA S.A.), 6.5% Eumulgin-48 (HENKEL), 10% Montanide-80 (SEPIC), 1.2% benzyl alcohol (ELF ATOCHEM/ATO) and 0.1 ml of triethanolamine (MERCK).

The antigenic phase was slowly added to the oily phase which was maintained under stirring. Once the addition of antigenic phase had been completed, stirring was continued for 10 minutes and the vaccine was stored at 4ºC until use.
Five different vaccines were prepared with diverse concentrations of recombinant antigen (rVP60) and with oily antigen, specifically:

| **Vaccine Reference** | **rVP60 Protein Concentrations (µg/1-ml dose)** |
|---|---|
| 0.5 W/O/W | 0.5 |
| 3 W/O/W | 3 |
| 10 W/O/W | 10 |
| 25 W/O/W | 25 |
| 50 W/O/W | 50 |

**B.** Additionally, other vaccines were formulated using Munokynin^{R} system as adjuvant (aluminum hydroxide and Quil-A, AMERICAN CYANAMID). The vaccines were formulated with two different concentrations of recombinant antigen (rVP60) and with Munokynin^{R} adjuvant, specifically:

| **Vaccine Reference** | **rVP60 Protein Concentrations (µg/1-ml dose)** |
|---|---|
| 0.5 W/W | 0.5 |
| 25 W/W | 25 |

### Example 10. Laboratory level efficacy and safety trials in rabbits with the vaccine.

### 10.1 Animals

New Zealand rabbits were used, 45 days old, and free of anti-RHDV antibodies evaluated by means of HAI technique (Example 1). The animals were distributed in cages in groups of two, so that in each cage there were two rabbits vaccinated with the same vaccinal antigen concentration.

### 10.2. Vaccination and revaccination

A trial was conducted with a total of 39 rabbits, of which:
a) 26 rabbits were vaccinated with one dose of 1 ml of the vaccines described in Example 9. Twenty-one days from the initial vaccination, they were revaccinated with a second dose of the same vaccine, except that for vaccine reference 3 W/O/W only 1 of the 4 rabbits initially vaccinated with this vaccine was revaccinated;
b) as a positive control, 4 rabbits were vaccinated and revaccinated with one dose of the Laboratorios Sobrino/Cyanamid commercial vaccine "CYLAP HVD^{R}"; and
c) 9 rabbits were left as sentinels or negative control, of which 4 were inoculated with a solution containing only oil adjuvant (reference W/O/W), 4 others with a suspension containing only Munokynin^{R} adjuvant (reference W/W), and 1 was not inoculated.

The animals were distributed in groups of 4 rabbits per dose and vaccine, except for vaccine reference 0.5 W/O/W for which there were only 2 rabbits. Vaccine was administered via intramuscular (IM) route in 2 animals of each group and via subcutaneous (SC) route in the other 2. The 2 animals in the group of vaccine reference 0.5 W/O/W were vaccinated via IM route.

From the start of vaccination to 35 days post vaccination, the following parameters were evaluated:

### A. Serological response by means of HAI technique

The animals were bled at Tₒ, T₁₄ , T₂₁ and T₃₅.
- Tₒ:: Bleeding previous to vaccination
- T₁₄:: Bleeding at 14 days post vaccination
- T₂₁:: Bleeding at 21 days post vaccination, coinciding with the time of revaccination
- T₃₅:: Bleeding at 35 days post vaccination and 14 days post revaccination

Table 1 shows the results of the hemagglutination inhibition titres at different times.

**Table 1:**

| Titres by HAI of rabbit sera corresponding to the efficacy trial with the recombinant vaccine against the disease caused by RHDV | | | | | | | |
|---|---|---|---|---|---|---|---|
| Rabbit no. | Vac . Ref. | Tₒ | T₁₄ | T₂₁ | T₃₅/Cₒ | C₆ | C₁₄ |
| 4552 (1) | 0.5 W/O/W | < 1/20 | < 1/20 | 1/20 | 1/5120 | 1/5120 | 1/40960 |
| 4553 (1) | | < 1/20 | < 1/20 | < 1/20 | 1/40 | 1/1280 | †* |
| 4557 (1) | 3 W/O/W | < 1/20 | < 1/20 | 1/40 | 1/160 | 1/160 | 1/81920 |
| 4558 (1) | | < 1/20 | < 1/20 | 1/20 | 1/40 | 1/640 | 1/2560 |
| 4559 (2) | | < 1/20 | 1/320 | 1/640 | 1/2560 | 1/10240 | 1/81920 |
| 4560 (2) | | < 1/20 | < 1/20 | 1/160 | 1/640 | 1/640 | 1/40960 |
| 4561 (1) | 10 W/O/W | < 1/20 | 1/320 | 1/640 | 1/2560 | 1/2560 | 1/40960 |
| 4562 (1) | | < 1/20 | 1/320 | 1/1280 | 1/5120 | 1/5120 | 1/40960 |
| 4563 (2) | | < 1/20 | 1/1280 | 1/5120 | 1/10240 | 1/10240 | 1/40960 |
| 4567 (2) | | < 1/20 | 1/1280 | ND | 1/1280 | 1/1280 | 1/40960 |
| 4594 (1) | 25 W/O/W | < 1/20 | 1/640 | 1/1280 | 1/640 | 1/1280 | 1/40960 |
| 4595 (1) | | < 1/20 | 1/640 | 1/1280 | 1/2560 | 1/2560 | 1/10290 |
| 4596 (2) | | < 1/20 | 1/40 | 1/640 | 1/1280 | 1/1280 | 1/40960 |
| 4597 (2) | | < 1/20 | 1/160 | 1/1280 | 1/1280 | 1/1280 | 1/40960 |
| 4598 (1) | 50 W/O/W | < 1/20 | 1/2560 | 1/10240 | 1/10240 | 1/20480 | 1/81920 |
| 4599 (1) | | < 1/20 | 1/5120 | 1/10240 | 1/20480 | 1/20480 | 1/81920 |
| 4591 (2) | | < 1/20 | 1/160 | 1/1280 | 1/10240 | 1/5120 | 1/81920 |
| 4592 (2) | | < 1/20 | 1/1280 | 1/1280 | 1/10240 | 1/5120 | 1/40960 |
| 4001 (1) | 0.5 W/W | < 1/20 | < 1/20 | 1/640 | 1/320 | 1/320 | 1/40960 |
| 4569 (1) | | < 1/20 | < 1/20 | 1/320 | 1/320 | 1/1280 | 1/40960 |
| 4002 (2) | | < 1/20 | 1/640 | 1/1280 | 1/5120 | 1/5120 | 1/81920 |
| 4570 (2) | | < 1/20 | < 1/20 | < 1/20 | 1/640 | 1/640 | 1/10240 |
| 4003 (1) | 25 W/W | < 1/20 | 1/320 | 1/1280 | †* | †* | †* |
| 4572 (1) | | < 1/20 | 1/640 | 1/320 | 1/1280 | †* | †* |
| 4004 (2) | | < 1/20 | 1/160 | 1/80 | 1/1280 | 1/640 | 1/5120 |
| 4576 (2) | | < 1/20 | 1/160 | 1/40 | 1/640 | †* | †* |
| 4593 (1) | CYLAP H VD^{R} | < 1/20 | 1/640 | 1/1280 | 1/10240 | 1/2560 | 1/40960 |
| 4006 (1) | | < 1/20 | 1/160 | 1/320 | 1/1280 | 1/1280 | 1/40960 |
| 4590 (2) | | < 1/20 | 1/320 | 1/1280 | 1/10240 | †* | †* |
| 4005 (2) | | < 1/20 | 1/320 | 1/320 | 1/10240 | 1/10240 | 1/81920 |
| 4600 (1) | W/O/W | < 1/20 | < 1/20 | < 1/20 | †* | †* | †* |
| 4577 (1) | | < 1/20 | < 1/20 | < 1/20 | < 1/20 | † | † |
| 4008 (2) | | < 1/20 | < 1/20 | < 1/20 | < 1/20 | † | † |
| 4580 (2) | | < 1/20 | < 1/20 | < 1/20 | <1/20 | † | † |
| 4014 (1) | W/W | < 1/20 | < 1/20 | < 1/20 | †* | †* | †* |
| 4579 (1) | | < 1/20 | < 1/20 | < 1/20 | < 1/20 | † | † |
| 4007 (2) | | < 1/20 | < 1/20 | < 1/20 | †* | †* | †* |
| 4583 (2) | | < 1/20 | < 1/20 | < 1/20 | 1/640 | † | † |
| 4013 | --- | < 1/20 | < 1/20 | < 1/20 | < 1/20 | † | † |

| | | | | | | | |
|---|---|---|---|---|---|---|---|
| (1): Intramuscular route | | | | | | | |
| (2): Subcutaneous route | | | | | | | |
| †: Death due to infection | | | | | | | |
| †*: Death due to bleeding ND: Not done | | | | | | | |

It can be observed that at 14 days post vaccination the animals gave positive serological response, except for those corresponding to the lower concentrations of recombinant antigen (e.g., 0.5 µg), irrespective of the adjuvant used. The animals vaccinated with a placebo did not give serological response detectable by HAI. As can be seen in the sera corresponding to the bleeding done on day 21, the antibody titres increased, and they started to be detectable even in those sera from vaccinations at low concentrations of recombinant antigen. After revaccination and prior to challenge, high antibody levels were observed even at the lower concentrations of recombinant antigen. Starting with the 10 µg dose, no important differences were observed regarding serological response.

### B. Local reaction at the site of injection

No significant local or general type reactions were observed in the rabbits during the period of vaccination.

The results obtained in sections A and B above allow to affirm that the vaccine comprising the RHDV recombinant VP60 protein -besides being a safe vaccine- induces seroconversion in rabbits, similarly to the seroconversion induced by the native vaccine (CYLAP HVD^{R}).

### 10.3. Efficacy of the vaccine

At 36 days post vaccination, all the rabbits were infected intranasally with a dose of 3.6x10⁴ LD₅₀ (lethal dose 50%) of RHDV. Challenge was done in security stables.

From the time of infection to 14 d.p.i., the following parameters were evaluated:

### A. Presence or absence of clinical signs characteristic of the disease, as well as the level of protection afforded by the vaccine

Out of a total of 32 infected rabbits -23 previously vaccinated with the recombinant antigen (rVP60), 3 with the commercial native vaccine, the remaining 6 were placebos (Table 1) -only the 6 sentinel animals had died by the 5th post-infection day manifesting typical signs of rabbit hemorrhagic disease, whereas the vaccinated animals resisted infection and were alive after 14 d.p.i., without any apparent clinical signs of the disease (Table 1). No apparent microscopic lung or liver lesions were observed in these animals, nor was RHDV detected in the liver of these animals. It must be pointed out that the rabbits (reference no. 4558, 4559 and 4560) vaccinated with a single dose of 3 µg of recombinant antigen (rVP60) also resisted infection -which indicates that a low dose of recombinant antigen can be sufficient to confer protection against the disease.

### B. Serological response (HAI)

The animals were bled at Cₒ, C₆ and C₁₄:
- Cₒ:: Bleeding prior to challenge
- C₆:: Bleeding at 6 days post challenge
- C₁₄:: Bleeding at 14 days post challenge

The results obtained from HAI titration of the post-infection sera from vaccinated animals appear in Table 1. At 6 d.p.i., the anti-RHDV antibody titres are very similar to the titres previous to challenge (T₃₅/Cₒ), whereas at 14 d.p.i., they are higher and, in general, more uniform. This increase in antibody titre is due to the booster effect of the virus used in the challenge.

### C. Presence or absence of virus in animals after challenge

Liver samples from the animals that died during challenge as well as from the animals sacrificed at 14 d.p.i. were analyzed to detect RHDV presence. Two animals from each vaccine group were selected from those in which possible liver lesions had been ovserved (one of them vaccinated IM route and the other SC route), and liver samples were collected. In both cases RHDV was isolated from these samples. For this purpose, a homogenate of the livers in PBS was prepared using an Ultraturrax (Example 1). The suspension produced was clarified by centrifugation at 1,600 g for 30 minutes. The supernatant produced was used to detect the presence or absence of RHDV by hemagglutination (Example 1). As Table 2 shows, RHDV was identified in the liver of the rabbits that died during challenge (control or sentinel rabbits), a fact which confirms the cause of death.

**Table 2**

| Post-challenge isolation and titration of RHDV from rabbit livers | | | |
|---|---|---|---|
| Rabbit no. | Vac. Ref. | Survival period (d.p.i) | HA* |
| 4552 | 0.5 W/O/W | ---- | Negative |
| 4553 | | 7 | Negative |
| 4557 | 3 W/O/W | ---- | Negative |
| 4559 | | ---- | Negative |
| 4561 | 10 W/O/W | ---- | Negative |
| 4563 | | ---- | Negative |
| 4594 | 25 W/O/W | ---- | Negative |
| 4596 | | ---- | Negative |
| 4598 | 50 W/O/W | ---- | Negative |
| 4591 | | ---- | Negative |
| 4001 | 0.5 W/W | ---- | Negative |
| 4002 | | ---- | Negative |
| 4004 | 25 W/W | ---- | Negative |
| 4593 | CYLAP HVD^{R} | ---- | Negative |
| 4005 | | ---- | Negative |
| 4577 | W/O/W | 4 | 65,536 |
| 4008 | | 4 | 32,768 |
| 4580 | | 3 | 32,768 |
| 4579 | W/W | 5 | 4** |
| 4583 | | 3 | 4,096 |
| 4013 | --- | 3 | 16,384 |

| | | | |
|---|---|---|---|
| * Hemagglutination (HAu/50 µl) | | | |
| ** Coccidia present in liver | | | |

Table 2 shows that a very low virus level was detected only in 1 control rabbit (ref. 4579) -attributable to a hepatic coccidiosis observed macroscopically in the animal. This may have been the cause of the low replication of RHDV, since part of the hepatic parenchyma had been destroyed. Virus was not detected in the liver of vaccinated animals sacrificed at 14 d.p.i. One rabbit (ref. 4553), vaccinated with the lowest concentration of recombinant antigen, died as a consequence of the bleeding done at 6 d.p.i., as demonstrated by the fact that RHDV was not isolated in liver (Table 2).

The results obtained allow to affirm that the recombinant vaccine containing recombinant VP60 protein is highly immunogenic and confers total protection -even at low doses- against rabbit hemorrhagic disease. No appreciable differences have been found between administration of the vaccine via intramuscular or subcutaneous routes.

Additionally, the recombinant vaccine is capable of preventing shedding (virus diffusion), as it has been possible to confirm that RHDV replication is avoided in target organs (liver) of vaccinated animals challenged with the virus.

### Example 11. Utilization of the recombinant VP60 protein in diagnostic methods and techniques

As mentioned in Example 7.3, recombinant VP60 protein can be used to detect specific anti-RHDV antibodies. This finding is useful to confirm infection in animals and determine the need for vaccination. The method followed was an indirect ELISA done in microtiter plates with 96 round-bottom wells. Briefly, the procedure was as follows: A plaque assay was done with 0.25 µg of recombinant antigen (rVP60) in carbonate buffer pH:9.6 per well, kept at 4ºC overnight. The sera object of the assay were added to the suitable dilution done with 0.1% PBS-Molico^{R} and incubated for 1 hour at room temperature. The plaques were washed and developed using ABTS as substrate (Example 8.3).

This procedure for the detection and quantitation of anti-RHDV antibodies was used as an example in the determination of ELISA titres of the rabbit sera corresponding to the efficacy test with recombinant vaccine. The results of the ELISA titration of anti-RHDV antibodies are shown in Table 3.

**Table 3:**

| Anti-RHDV antibodies determined by ELISA | | | | | | | |
|---|---|---|---|---|---|---|---|
| Rabbit no. | Vac. Ref. | Tₒ | T₁₄ | T₂₁ | T₃₅/Cₒ | C₆ | C₁₄ |
| 4552 (1) | 0.5 W/O/W | < 1/20 | <1/100 | 1/200 | 1/12800 | 1/80000 | 1/160000 |
| 4553 (1) | | < 1/20 | < 1/100 | < 1/100 | 1/400 | 1/10000 | †* |
| 4557(1) | 3 W/O/W | < 1/20 | < 1/100 | 1/100 | 1/6400 | 1/20000 | 1/80000 |
| 4558 (1) | | < 1/20 | < 1/100 | <1/100 | 1/1600 | 1/5000 | 1/40000 |
| 4559 (2) | | < 1/20 | 1/100 | 1/3200 | 1/3200 | 1/40000 | 1/320000 |
| 4560 (2) | | < 1/20 | < 1/100 | 1/800 | 1/1600 | 1/10000 | 1/320000 |
| 4561 (1) | 10 W/O/W | < 1/20 | 1/200 | 1/3200 | 1/40000 | 1/80000 | 1/320000 |
| 4562 (1) | | < 1/20 | 1/400 | 1/3200 | 1/20000 | 1/80000 | 1/640000 |
| 4563 (2) | | < 1/20 | 1/1600 | 1/12800 | 1/320000 | 1/160000 | 1/320000 |
| 4567 (2) | | < 1/20 | 1/3200 | 1/12800 | 1/320000 | 1/80000 | 1/160000 |
| 4594 (1) | 25 W/O/W | < 1/20 | 1/3200 | 1/6400 | 1/40000 | 1/40000 | 1/160000 |
| 4595 (1) | | < 1/20 | 1/1600 | 1/12800 | 1/80000 | 1/40000 | 1/160000 |
| 4596 (2) | | < 1/20 | 1/1600 | 1/3200 | 1/40000 | 1/40000 | 1/160000 |
| 4597 (2) | | 1/160 | 1/6400 | 1/12800 | 1/40000 | 1/40000 | >1/10⁶ |
| 4598 (1) | 50 W/O/W | < 1/20 | 1/6400 | 1/12800 | 1/80000 | 1/320000 | 1/320000 |
| 4599 (1) | | < 1/20 | 1/6400 | 1/12800 | 1/80000 | 1/160000 | 1/160000 |
| 4591 (2) | | < 1/20 | 1/3200 | 1/12800 | 1/80000 | 1/80000 | 1/80000 |
| 4592 (2) | | < 1/20 | 1/6400 | 1/12800 | 1/40000 | 1/40000 | 1/160000 |
| 4001 (1) | 0.5 W/W | < 1/20 | 1/800 | 1/1600 | 1/6400 | 1/10000 | 1/160000 |
| 4569 (1) | | 1/320 | 1/400 | 1/800 | 1/6400 | 1/20000 | 1/320000 |
| 4002 (2) | | < 1/20 | 1/1600 | 1/1600 | 1/20000 | 1/80000 | 1/160000 |
| 4570 (2) | | < 1/20 | < 1/100 | < 1/100 | 1/200 | 1/10000 | 1/40000 |
| 4003 (1) | 25 W/W | < 1/20 | 1/6400 | 1/6400 | †* | †* | †* |
| 4572 (1) | | 1/160 | 1/400 | 1/800 | 1/12800 | †* | †* |
| 4004 (2) | | < 1/20 | 1/800 | 1/1600 | 1/6400 | 1/10000 | 1/80000 |
| 4576 (2) | | < 1/20 | 1/100 | 1/200 | 1/6400 | †* | †* |
| 4593 (1) | CYLAP HVD^{R} | < 1/20 | 1/1600 | 1/12800 | 1/40000 | 1/40000 | 1/160000 |
| 4006 (1) | | < 1/20 | 1/800 | 1/3200 | 1/40000 | 1/80000 | 1/160000 |
| 4590 (2) | | < 1/20 | 1/200 | 1/1600 | 1/80000 | †* | †* |
| 4005 (1) | | < 1/20 | 1/1600 | 1/6400 | 1/80000 | 1/160000 | 1/320000 |
| 4600 (1) | W/O/W | 1/160 | 1/200 | 1/200 | †* | †* | †* |
| 4577 (2) | | < 1/20 | < 1/100 | 1/100 | 1/400 | † | † |
| 4008 (2) | | < 1/20 | 1/200 | 1/400 | 1/100 | † | † |
| 4580 (1) | | < 1/20 | < 1/100 | 1/200 | 1/400 | † | † |
| 4014 (1) | W/W | 1/80 | 1/400 | 1/800 | †* | †* | †* |
| 4579 (2) | | < 1/20 | < 1/100 | < 1/100 | < 1/100 | † | † |
| 4007 (1) | | < 1/20 | 1/100 | < 1/100 | †* | †* | †* |
| 4583 (2) | | < 1/20 | < 1/100 | < 1/100 | <1/100 | † | † |
| 4013 (-) | --- | < 1/20 | < 1/100 | < 1/100 | < 1/100 | † | † |

| | | | | | | | |
|---|---|---|---|---|---|---|---|
| (1): Intramuscular route | | | | | | | |
| (2): Subcutaneous route | | | | | | | |
| †: Death due to infection | | | | | | | |
| †*: Death due to bleeding | | | | | | | |

In general, the correlation between the titres obtained by HAI and ELISA is quite good, although the sensitivity of the ELISA method is 10 times higher and the titres obtained are between 5 and 20 times higher.

It is convenient to emphasize that appreciable antibody titres were detected by ELISA but not by HAI in four rabbits (ref. 4597, 4569, 4572 and 4600) - a fact that might indicate residual maternal immunity which, however, does not seem to affect the induction of antibodies. This result suggests that the vaccine could be efficacious even in the presence of maternal antibodies.

### Example 12. Vaccination via oral route - efficacy and safety trial with the vaccine

Vaccines -capable of protecting rabbits against challenge with RHDV- were prepared in the form of an aqueous suspension, following the procedure described below:

A 300 ml suspension culture of Sf9 cells was infected with recombinant baculovirus AcNPV-RHD710 at multliplicity of infection (m.o.i.) of 1 pfu/cell. The cells were collected at 72 h.p.i. and processed as described above in Example 7 until the final step of purification of the recombinant VP60 protein with ammonium sulfate at 30%. This sample was analyzed in SDS-polyacrilamide gels at 9% and immunoblot using anti-RHDV serum. A majoritarian band (>80%) with molecular weight of approximately 60 KDa was observed, together with two smaller bands (43-60 KDa) copurifying with VP60, although -not having been detected with anti-RHDV serum- they are not virus specific.

Recombinant VP60 protein concentration in the preparation was 2 mg/ml, determined by Lowry and SDS-gel with Coomasie stain.
**A.** A vaccine was formulated as follows: Recombinant VP60 protein was diluted in PBS pH 7.2 so that 1 ml of the dilution (1 dose) would contain 3 µg of recombinant VP60 protein. This solution was inactivated with binary ethylenimine at final concentration of 5mM at 37ºC for 72 hours.
**B.** Additionally, another vaccine was formulated using the same antigenic solution at a concentration of 3 µg of recombinant VP60 per dose of 1 ml, but not inactivated.

### - Vaccines tested: inactivated vaccine vaccine not inactivated

### - Animals

New Zealand rabbits were used, 60 days old, and free of anti-RHDV antibodies evaluated by means of HAI technique (Example 1). The animals were distributed in cages.

### - Vaccination and revaccination

A trial was conducted with a total of 15 rabbits, of which:
a) 5 rabbits were vaccinated with one dose of 1 ml of inactivated vaccine. Twenty-one days from the initial vaccination, they were revaccinated with a second dose of the same vaccine [Group 15, Table 4].
b) 5 rabbits were vaccinated and revaccinated with one dose of non-inactivated vaccine [Group 16, Table 4].
c) 5 rabbits, left as sentinels or negative control, were inoculated with PBS [Group 17, Table 4].

Vaccine was administered in the rabbits via oral route, depositing 1 ml of the aqueous phase in the mouth.

From the start of vaccination to 35 days post vaccination, the following parameters were evaluated:

### A. Serological response by means of HAI technique

The animals were bled at T₋₄, T₂₁ and T₃₅, T₄₃.
- T₋₄:: Bleeding at 4 days previous to vaccination
- T₂₁:: Bleeding at 21 days post vaccination, coinciding with the time of revaccination
- T₃₅:: Bleeding at 35 days post vaccination and 14 days post revaccination
- T₄₃:: Bleeding at 43 days post vaccination and 22 days post revaccination

Table 4 shows the results of the hemagglutination inhibition titres at different times.

**Table 4:**

| Titres by HAI of rabbit sera corresponding to the efficacy trial (oral vaccination) with the recombinant vaccine against the disease caused by RHDV | | | | | | | |
|---|---|---|---|---|---|---|---|
| Group | Animal NO. | T₋₄ | T₂₁ | T₃₅ | T₄₃ | | Virus in liver HA titre |
| | | | | | Survival D8 post challenge | | |
| 15 | 407 | <1/20 | <1/20 | <1/20 | 1/320 | Yes | No,neg. |
| | 401 | <1/20 | 1/160 | 1/640 | 1/2560 | Yes | No,neg. |
| | 413 | <1/20 | 1/20-1/40 | ≤1/20 | 1/1280 | Yes | No,neg. |
| | 411 | <1/20 | 1/80-1/160 | 1/40 | 1/640 | Yes | Y 1/8 |
| | 405 | <1/20 | <1/20 | <1/20 | - | † | Y 1/8192 |
| 16 | 402 | <1/20 | <1/20 | <1/20 | - | † | Y 1/8192 |
| | 415 | <1/20 | 1/80-1/160 | 1/160 | 1/1280 | Yes | Y 1/4 |
| | 409 | <1/20 | <1/20 | ≤1/20 | - | † | Y 1/4096 |
| | 408 | <1/20 | <1/20 | * | - | * | * |
| | 410 | <1/20 | <1/20 | <1/20 | - | † | Y 1/64 |
| 17 | 414 | <1/20 | <1/20 | * | - | * | * |
| | 403 | <1/20 | <1/20 | <1/20 | - | † | Y 1/8192 |
| | 404 | <1/20 | <1/20 | <1/20 | - | † | Y 1/16384 |
| | 412 | <1/20 | <1/20 | <1/20 | - | † | Y 1/4096 |
| | 406 | <1/20 | <1/20 | <1/20 | - | † | Y 1/8192 |

| | | | | | | | |
|---|---|---|---|---|---|---|---|
| *: Death due to bleeding | | | | | | | |
| †: Death due to infection Y: Yes **Neg: Negative** | | | | | | | |

It can be observed that at 21 days post vaccination, three of the five animals vaccinated with inactivated vaccine (ref. no. 401, 413 and 411) gave positive serological response. Of the animals vaccinated with non-inactivated vaccine, only one (no. 415) seroconverted.

### B. General reaction

No significant local or general type reactions were observed in the rabbits during the period of vaccination.

The results obtained in Table 4 allow to affirm that the vaccine comprising RHDV recombinant VP60 protein induces seroconversion in rabbits, although at lower potency than the vaccine administered via subcutaneous route.

### - Efficacy of the vaccine

At 35 days post vaccination, all the rabbits were infected intranasally with a dose of 3.6x10⁴ LD₅₀ (lethal dose 50%) of RHDV. Challenge was done in security stables.

From the time of infection to 8 d.p.i., the following parameters were evaluated:

### A. Presence or absence of clinical signs characteristic of the disease, as well as the level of protection afforded by the vaccine

Of the total of 15 infected rabbits, 5 had been vaccinated with inactivated recombinant antigen (rVP60), 5 with the non-inactivated recombinant antigen and the remaining 5 were placebos (Table 4). Of the sentinel animals, 100% had died by the 8th post-infection day, manifesting typical signs of rabbit hemorrhagic disease. Of the animals vaccinated with the non-inactivated antigen, 3 out of 4 died. Of the 5 animals vaccinated with inactivated vaccine, 1 died.

### B. Presence or absence of virus in animals after challenge

Samples of livers from animals that had died before the 8th day post infection as well as from animals sacrificed on the 8th day post infection were obtained, according to the method mentioned in Example 1, in order to detect and quantify presence of RHDV. As can be seen in Table 4, RHDV concentrations in the livers from the sacrificed animals were practically nil (corresponding to vaccinated animals), whereas in the livers from the animals dead before 8 d.p.i., high concentrations of RHDV were detected. Therefore, these vaccines, like the IM and SC vaccines (Examples 9 and 10), not only prevent clinical signs but, furthermore, avoid RHDV replication at the level of the analyzed organ (liver).

The results obtained allow to affirm that the recombinant vaccine containing inactivated recombinant VP60 protein is immunogenic and confers protection against rabbit hemorrhagic disease after vaccination via oral route.

### DEPOSIT OF MICROORGANISMS

The recombinant baculovirus denominated AcNPV RHDV-710 was deposited at the European Collection of Animal Cell Cultures (ECACC), Porton Down, Salisbury, Whiltshire SP4 OJG, United Kingdom on 18th May 1994, with accession number V94051819.

The intention is to avoid having the scope of this invention limited by the recombinant baculovirus that has been deposited, since the deposit effected is intended only as an illustrative example of a recombinant baculovirus suitable for the realization of this invention.

## Claims

1. An empty capsid consisting of recombinant rabbit hemorrhagic disease virus (RHDV) VP60 protein.

2. A capsid according to claim 1, consisting of RHDV recombinant VP60 protein expressed in permissive insect cells infected with a recombinant baculovirus having the DNA sequence coding for RHDV VP60 protein.

3. A capsid according to anyone of claims 1 or 2, said capsid being antigenically and immunogenically analogous to RHDV.

4. A capsid according to anyone of the preceding claims, wherein said RHDV recombinant VP60 protein comprises a modified RHDV VP60 protein, said modified RHDV VP60 protein consisting of the RHDV native VP60 protein wherein:
(i) the amino acid residues at sites +2 and +3 of the amino acid sequence of RHDV native VP60 protein as shown in Figure 2 have been substituted by two other amino acid residues, and
(ii) an additional amino acid sequence having up to five amino acid residues is located at the amino terminal end of the recombinant VP60 protein, said additional amino acid sequence being not present in the native VP60 protein.

5. A capsid according to claim 4, wherein said additional amino acid sequence located at the amino terminal end of the recombinant VP60 protein has the following sequence:

Ala-Cys-Ile-Asp-Arg

6. A rabbit hemorrhagic disease virus (RHDV) recombinant VP60 protein, said RHDV recombinant VP60 protein comprising a modified RHDV VP60 protein, said modified RHDV VP60 protein consisting of the RHDV native VP60 protein wherein:
(i) the amino acid residues at sites +2 and +3 of the amino acid sequence of RHDV native VP60 protein as shown in Figure 2, have been substituted by two other amino acid residues, and
(ii) an additional amino acid sequence having up to five amino acid residues is located at the amino terminal end of the recombinant VP60 protein, said additional amino acid sequence being not present in the native VP60 protein.

7. Recombinant VP60 protein according to claim 6, wherein said additional amino acid sequence located at the amino terminal end of the recombinant VP60 protein has the following sequence:

Ala-Cys-Ile-Asp-Arg

8. Recombinant VP60 protein according to claim 6, said recombinant VP60 protein being antigenically and immunogenically analogous to RHDV native VP60 protein.

9. A vaccine suitable for the vaccination and protection of rabbits against rabbit hemorrhagic disease (RHD), comprising:
(i) an immunologically suitable quantity of an antigen comprising:
i.a) a capsid as per any one of claims 1 to 5; or
i.b) a RHDV recombinant VP60 protein as per any one of claims 6 to 8; and
(ii) a suitable carrier or adjuvant.

10. Vaccine according to claim 9, wherein said adjuvant is an oil adjuvant based on mineral oils, glycerides and fatty ether-acid derivatives.

11. Vaccine according to claim 9, in the form of an emulsion W/O or O/W, or in the form of a double emulsion W/O/W.

12. Vaccine according to claim 9, wherein said adjuvant is a synthetic adjuvant.

13. Vaccine according to claim 9, wherein said adjuvant is an aqueous adjuvant selected from the group consisting of aluminum hydroxide, a suspension of alumina gels, Quil A and mixtures thereof.

14. vaccine according to claim 9, wherein said adjuvant is selected from the group consisting of MDP (murarnyl dipetide), ISCOM (Immuno Stimulant Complex) or liposomes.

15. Vaccine according to claim 9, said vaccine being suitable for administration via Intramuscular, Subcutaneous, Intradermal or Oral routes.

16. Vaccine according to claim 9, wherein the antigen is presented in the form of a solution or suspension with an adjuvant or a diluent.

17. Vaccine according to claim 9, said vaccine being in a freeze-dried form susceptible of being reconstituted with a diluent, adjuvant or another aqueous or oily rabbit vaccine.

18. Vaccine according to claim 9, for protecting wild rabbits, farm rabbits and pet rabbits against RHDV.

19. A bi- or multivalent vaccine capable of preventing rabbit hemorrhagic disease (RHD) in rabbits and other infection or infections of rabbits, comprising:
i) an immunologically suitable quantity of an antigen comprising:
i.a) a capsid as per any one of claims 1 to 5; or
i.b) a RHDV recombinant VP60 protein as per any one of claims 6 to 8;
ii) one or more additional antigen(s), said additional antigen(s) being related to rabbit pathogens; and
iii) a suitable carrier or adjuvant.

20. Vaccine according to claim 19, wherein said pathogens are selected from the group consisting of myxomatosis virus, Shope's Fibroma virus, *Bordetella bronchiseptica, Pasteurella* spp., *Clostridia* spp., *Salmonella* spp., *Staphylococcus* spp. and *Haemophilus* spp.

21. Use of a capsid as per any one of claims 1 to 5, or a RHDV recombinant VP60 protein as per any one of claims 6 to 8, in the manufacture of a vaccine to protect rabbits from the infection caused by RHDV.

22. A method for detecting the presence of antibodies that specifically recognize rabbit hemorrhagic disease virus (RHDV) in a biological sample from a rabbit, which comprises contacting said biological sample with empty capsids as per any one of claims 1 to 5, or with RHDV recombinant VP60 proteins as per any one of claims 6 to 8, and detecting the formation of an antigen-antibody complex.

23. A diagnostic kit for detecting the presence of antibodies that specifically recognize rabbit hemorrhagic disease virus (RHDV) in a biological sample from a rabbit, comprising an empty capsid as per any one of claims 1 to 5, or a RHDV recombinant VP60 protein as per any one of claims 6 to 8, and suitable detection means.

24. A recombinant baculovirus, denominated AcNPV RHDV-710, deposited at the ECACC with accession number V94051819.

## Patentansprüche

1. Capsid, bestehend aus dem rekombinanten Rabbit Hemorrhagic Disease Virus (RHDV) VP60 Protein.

2. Capsid gemäß Anspruch 1, bestehend aus RHDV rekombinantem VP60 Protein, exprimiert in permissiven Insektenzellen, infiziert mit einem rekombinanten Baculovirus mit der DNA-Sequenz, welche für RHDV VP60 Protein kodiert.

3. Capsid gemäß einem der Ansprüche 1 oder 2, wobei besagtes Capsid antigen und immunogen analog zu RHDV ist.

4. Capsid gemäß einem der vorhergehenden Ansprüche, wobei besagtes RHDV rekombinantes VP60 Protein ein modifiziertes RHDV VP60 Protein umfasst, wobei besagtes modifiziertes RHDV VP60 Protein aus dem RHDV nativen VP60 Protein besteht, worin:
(i) die Aminosäurereste an Stellen +2 und +3 der Aminosäuresequenz von RHDV nativem VP60 Protein wie in Figur 2 gezeigt durch zwei andere Aminosäurereste substituiert worden sind und
(ii) sich eine zusätzliche Aminosäuresequenz mit bis zu fünf Aminosäureresten an dem Amino-terminalen Ende des rekombinanten VP60 Proteins befindet, wobei besagte zusätzliche Aminosäuresequenz im nativen VP60 Protein nicht vorhanden ist.

5. Capsid gemäß Anspruch 4, worin besagte zusätzliche Aminosäuresequenz, welche sich an dem Amino-terminalen Ende des rekombinanten VP60 Proteins befindet, die folgende Sequenz: Ala-Cys-Ile-Asp-Arg hat.

6. Rabbit Hemorrhagic Disease Virus (RHDV) rekombinantes VP60 Protein, wobei besagtes RHDV rekombinantes VP60 Protein ein modifiziertes RHDV VP60 Protein umfasst, wobei besagtes modifiziertes RHDV VP60 Protein aus dem RHDV nativen VP60 Protein besteht, worin:
(i) die Aminosäurereste an Stellen +2 und +3 der Aminosäuresequenz von RHDV nativem VP60 Protein wie in Figur 2 gezeigt durch zwei andere Aminosäurereste substituiert worden sind und
(ii) sich eine zusätzliche Aminosäuresequenz mit bis zu fünf Aminosäureresten an dem Amino-terminalen Ende des rekombinanten VP60 Proteins befindet, wobei besagte zusätzliche Aminosäuresequenz im nativen VP60 Protein nicht vorhanden ist.

7. Rekombinantes VP60 Protein gemäß Anspruch 6, worin besagte zusätzliche Aminosäuresequenz, welche sich an dem Amino-terminalen Ende des rekombinanten VP60 Proteins befindet, die folgende Sequenz: Ala-Cys-Ile-Asp-Arg hat.

8. Rekombinantes VP60 Protein gemäß Anspruch 6, wobei besagtes rekombinantes VP60 Protein antigen und immunogen analog zu RHDV nativem VP60 Protein ist.

9. Impfstoff, geeignet zur Impfung und zum Schutz von Kaninchen gegenüber Rabbit Hemorrhagic Disease (RHD), welcher umfasst:
(i) eine immunologisch geeignete Menge eines Antigens, umfassend:
i.a) ein Capsid wie nach einem der Ansprüche 1 bis 5; oder
i.b) ein RHDV rekombinantes VP60 Protein wie nach einem der Ansprüche 6 bis 8; und
(ii) einen geeigneten Träger oder Adjuvans.

10. Impfstoff gemäß Anspruch 9, worin besagtes Adjuvans ein Öl-Adjuvans, basierend auf Mineralölen, Glyceriden und Fettsäureether Derivaten ist.

11. Impfstoff gemäß Anspruch 9 in Form einer Emulsion W/O oder O/W oder in Form einer Doppelemulsion W/O/W.

12. Impfstoff gemäß Anspruch 9, worin besagtes Adjuvans ein synthetisches Adjuvans ist.

13. Impfstoff gemäß Anspruch 9, worin besagtes Adjuvans ein wässeriges Adjuvans ist, ausgewählt aus der Gruppe bestehend aus Aluminiumhydroxid, einer Suspension aus Tonerdegels, Quil A und Gemischen daraus.

14. Impfstoff gemäß Anspruch 9, worin besagtes Adjuvans ausgewählt wird aus der Gruppe bestehend aus MDP (Muramyldipeptid), ISCOM (Immuno Stimulant Complex) oder Liposomen.

15. Impfstoff gemäß Anspruch 9, wobei besagter Impfstoff zur Verabreichung auf intramuskulären, subkutanen, intradermalen oder oralen Wegen geeignet ist.

16. Impfstoff gemäß Anspruch 9, wobei das Antigen in Form einer Lösung oder Suspension mit einem Hilfsstoff oder Verdünnungsmittel vorhanden ist.

17. Impfstoff gemäß Anspruch 9, wobei besagter Impfstoff eine gefriergetrocknete Form hat, welche mit einem Verdünnungsmittel, Adjuvans oder einem anderen wässerigen oder öligen Kaninchen-Impfstoff rekonstituiert werden kann.

18. Impfstoff gemäß Anspruch 9 zum Schutz von wilden Kaninchen, Hof-Kaninchen und Haustier-Kaninchen vor RHDV.

19. Zwei- oder mehrwertiger Impfstoff, welcher in der Lage ist, Rabbit Hemorrhagic Disease (RHD) in Kaninchen und andere Infektion oder Infektionen von Kaninchen zu verhindern, welcher umfasst:
i) eine immunologisch geeignete Menge eines Antigens, welches umfasst:
i.a) ein Capsid wie nach einem der Ansprüche 1 bis 5; oder
1.b) ein RHDV rekombinantes VP60 Protein wie nach einem der Ansprüche 6 bis 8;
ii) ein oder mehrere zusätzliche(s) Antigen(e), wobei besagte(s) Antigen(e) mit Kaninchen-Pathogenen in Bezug stehen; und
iii) einen geeigneten Träger oder Adjuvans.

20. Impfstoff gemäß Anspruch 19, worin besagte Pathogene ausgewählt werden aus der Gruppe bestehend aus Myxomatose-Virus, Shope's Fibroma Virus, *Bordetella bronchiseptica, Pasteurella* spp., *Clostridia* spp., *Salmonella* spp., *Staphylococcus* spp. und *Haemophilus* spp.

21. Verwendung eines Capsids wie nach einem der Ansprüche 1 bis 5 oder eines RHDV rekombinanten VP60 Proteins wie nach einem der Ansprüche 6 bis 8 bei der Herstellung eines Impfstoffs, um Kaninchen vor durch RHDV verursachte Infektion zu schützen.

22. Verfahren zum Nachweisen der Gegenwart von Antikörpern, die speziell Rabbit Hemorrhagic Disease Virus (RHDV) in einer biologischen Probe von einem Kaninchen erkennen, welches umfasst:
Kontaktieren besagter biologischer Probe mit leeren Capsiden wie nach einem der Ansprüche 1 bis 5 oder mit RHDV rekombinanten VP60 Proteinen wie nach einem der Ansprüche 6 bis 8 und Nachweisen der Bildung eines Antigen-Antikörper-Komplexes.

23. Diagnostisches Kit zum Nachweisen der Gegenwart von Antikörpern, die speziell Rabbit Hemorrhagic Disease Virus (RHDV) in einer biologischen Probe von einem Kaninchen erkennen, welches ein leeres Capsid wie nach einem der Ansprüche 1 bis 5 oder ein RHDV rekombinantes VP60 Protein wie nach einem der Ansprüche 6 bis 8 und geeignete Nachweismittel umfasst.

24. Rekombinantes Baculovirus mit der Bezeichnung AcNPV RHDV-710, hinterlegt bei der ECACC mit Hinterlegungsnummer V94051819.

## Revendications

1. Capside vide comprenant une protéine VP60 recombinante du virus de la maladie hémorragique du lapin (RHDV).

2. Capside suivant la revendication 1, comprenant une protéine VP60 recombinante du RHDV exprimée dans des cellules permissives d'insecte infectées par un baculovirus recombinant présentant la séquence d'ADN codant pour la protéine VP60 du RHDV.

3. Capside suivant l'une quelconque des revendications 1 ou 2, ladite capside étant analogue sur le plan antigénique et sur le plan immunogène au RHDV.

4. Capside suivant l'une quelconque des revendications précédentes, dans laquelle ladite protéine VP60 recombinante du RHDV comprend une protéine VP60 modifiée du RHDV, ladite protéine VP60 modifiée du RHDV comprenant la protéine VP60 native du RHDV, dans laquelle :
(i) les résidus acides aminés aux sites +2 et +3 de la séquence d'acides aminés de la protéine VP60 native du RHDV telle que représentée à la Fig. 2 sont remplacés par deux autres résidus acides aminés, et
(ii) une séquence supplémentaire d'acides aminés présentant jusqu'à cinq résidus acides aminés est localisée à l'extrémité amino de la protéine VP60 recombinante, ladite séquence supplémentaire d'acides aminés n'étant pas présente dans la protéine VP60 native.

5. Capside suivant la revendication 4, dans laquelle ladite séquence supplémentaire d'acides aminés localisée à l'extrémité amino de la protéine VP60 recombinante présente la séquence suivante :

Ala-Cys-Ile-Asp-Arg

6. Protéine VP60 recombinante du virus de la maladie hémorragique du lapin (RHDV), ladite protéine VP60 recombinante du RHDV comprenant une protéine VP60 modifiée du RHDV, ladite protéine VP60 modifiée du RHDV comprenant la protéine VP60 native du RHDV, dans laquelle :
(i) les résidus acides aminés aux sites +2 et +3 de la séquence d'acides aminés de la protéine VP60 native du RHDV telle que présentée à la Fig. 2 sont remplacés par deux autres résidus acides aminés, et
(ii) une séquence supplémentaire d'acides aminés présentant jusqu'à cinq résidus acides aminés est localisée à l'extrémité amino de la protéine VP60 recombinante, ladite séquence supplémentaire d'acides aminés n'étant pas présente dans la protéine VP60 native.

7. Protéine VP60 recombinante suivant la revendication 6, dans laquelle ladite séquence supplémentaire d'acides aminés localisée à l'extrémité amino de la protéine VP60 recombinante présente la séquence suivante :

Ala-Cys-Ile-Asp-Arg

8. Protéine VP60 recombinante suivant la revendication 6, ladite protéine VP60 recombinante étant analogue sur le plan antigénique et sur le plan immunogène à la protéine VP60 native du RHDV.

9. Vaccin approprié pour la vaccination et la protection de lapins contre la maladie hémorragique du lapin (RHD), comprenant :
(i) une quantité appropriée sur le plan immunologique d'un antigène comprenant :
i.a) une capside telle que suivant l'une quelconque des revendications 1 à 5; ou
i.b) une protéine VP60 recombinante du RHDV telle que suivant l'une quelconque des revendications 6 à 8; et
(ii) un vecteur ou adjuvant approprié.

10. Vaccin suivant la revendication 9, dans lequel ledit adjuvant est un adjuvant huileux à base d'huiles minérales, de glycérides et de dérivés éther d'acide gras.

11. Vaccin suivant la revendication 9, sous forme d'une émulsion eau/huile ou huile/eau, ou sous forme d'une émulsion double eau/huile/eau.

12. Vaccin suivant la revendication 9, dans lequel ledit adjuvant est un adjuvant synthétique.

13. Vaccin suivant la revendication 9, dans lequel ledit adjuvant est un adjuvant aqueux sélectionné parmi le groupe comprenant de l'hydroxyde d'aluminium, une suspension de gels d'alumine, du Quil A et des mélanges de ceux-ci.

14. Vaccin suivant la revendication 9, dans lequel ledit adjuvant est sélectionné parmi le groupe comprenant du MDP (muramyldipeptide), de l'ISCOM (complexe immunostimulant) ou des liposomes.

15. Vaccin suivant la revendication 9, dans lequel ledit vaccin est approprié pour une administration par voie intramusculaire, sous-cutanée, percutanée ou orale.

16. Vaccin suivant la revendication 9, dans lequel l'antigène est présenté sous forme d'une solution ou d'une suspension avec un adjuvant ou un diluant.

17. Vaccin suivant la revendication 9, ledit vaccin étant sous forme lyophilisée susceptible d'être reconstituée avec un diluant, un adjuvant ou un autre vaccin aqueux ou huileux pour lapin.

18. Vaccin suivant la revendication 9, pour la protection de lapins sauvages, de lapins d'élevage et de lapins domestiques contre le RHDV.

19. Vaccin bivalent ou multivalent pouvant empêcher l'apparition de la maladie hémorragique du lapin (RHD) chez des lapins et une autre infection ou d'autres infections de lapins, comprenant :
i) une quantité appropriée sur le plan immunologique d'un antigène comprenant :
i.a) une capside telle que suivant l'une quelconque des revendications 1 à 5; ou
i.b) une protéine VP60 recombinante du RHDV telle que suivant l'une quelconque des revendications 6 à 8;
ii) un ou plusieurs antigène(s) supplémentaire(s), le(s)dit(s) antigène(s) supplémentaire(s) étant apparenté(s) à des pathogènes du lapin; et
iii) un vecteur ou adjuvant approprié.

20. Vaccin suivant la revendication 19, dans lequel lesdits pathogènes sont sélectionnés parmi le groupe comprenant le virus de la dégénérescence myxomateuse, le virus du fibrome de Shope, *Bordetella* *bronchiseptica, Pasteurella* species, *Clostridia* species, *Salmonella species, Staphylococcus species* et *Haemophilus* species.

21. Utilisation d'une capside telle que suivant l'une quelconque des revendications 1 à 5 ou d'une protéine VP60 recombinante du RHDV telle que suivant l'une quelconque des revendications 6 à 8, dans la fabrication d'un vaccin pour protéger des lapins contre une infection provoquée par le RHDV.

22. Procédé de détection de la présence d'anticorps qui reconnaissent spécifiquement le virus de la maladie hémorragique du lapin (RHDV) dans un échantillon biologique provenant d'un lapin, qui comprend la mise en contact dudit échantillon biologique avec des capsides vides telles que suivant l'une quelconque des revendications 1 à 5 ou avec des protéines VP60 recombinantes du RHDV telles que suivant l'une quelconque des revendications 6 à 8 et la détection de la formation d'un complexe antigène-anticorps.

23. Kit de diagnostic pour la détection de la présence d'anticorps qui reconnaissent spécifiquement un virus de la maladie hémorragique du lapin (RHDV) dans un échantillon biologique provenant d'un lapin, comprenant une capside vide telle que suivant l'une quelconque des revendications 1 à 5 ou une protéine VP60 recombinante du RHDV telle que suivant l'une quelconque des revendications 6 à 8 et un moyen approprié de détection.

24. Baculovirus recombinant nommé AcNPV RHDV-710, déposé à l'ECACC sous le numéro d'enregistrement V94 051 819.
